(19) Europäisches Patentamt
European Patent Office
Office européen des brevets

(11) EP 1 508 557 A1

(12) **EUROPEAN PATENT APPLICATION**
published in accordance with Art. 158(3) EPC

(43) Date of publication:
**23.02.2005 Bulletin 2005/08**

(21) Application number: **03730606.5**

(22) Date of filing: **23.05.2003**

(51) Int Cl.[7]: **C07C 25/02**, C07C 25/125, C07C 17/12, C07B 61/00

(86) International application number:
**PCT/JP2003/006464**

(87) International publication number:
**WO 2003/099748 (04.12.2003 Gazette 2003/49)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **27.05.2002 JP 2002152458**
**24.07.2002 JP 2002214927**

(71) Applicant: **TORAY INDUSTRIES, INC.**
**Tokyo 103-8666 (JP)**

(72) Inventors:
- **KATO, Hajime**
  **Chita-gun, Aichi 470-2103 (JP)**
- **UCHIYAMA, Naoyuki**
  **Nagoya-shi, Aichi 457-0086 (JP)**
- **MINOMIYA, Eiichi**
  **Okazaki-shi, Aichi 444-3505 (JP)**
- **YOSHIKAWA, Masahito**
  **Nagoya-shi, Aichi 458-0033 (JP)**
- **MIYAMOTO, Tohru**
  **Yokohama-shi, Kanagawa 236-0052 (JP)**
- **HIGUCHI, Kazuyoshi**
  **Ohbu-shi, Aichi 474-0072 (JP)**

(74) Representative: **Kador & Partner**
**Corneliusstrasse 15**
**80469 München (DE)**

# (54) PROCESS FOR HALOGENATION OF BENZENE AND BENZENE DERIVATIVES

(57) In a process of halogenation of benzene or benzene derivatives, di-substituted halobenzene derivatives having para-aromatic compounds or tri-substituted halobenzene derivatives having 1,2,4-substituted aromatic compounds are selectively produced. In halogenation of benzene or benzene derivatives, a fluorine-containing zeolite catalyst such as L-type zeolite, or a zeolite catalyst having the crystal size of at most 100 nm is used. The reaction is preferably effected in the presence of a solvent, and the solvent is preferably a halogenated compound.

Fig. 1

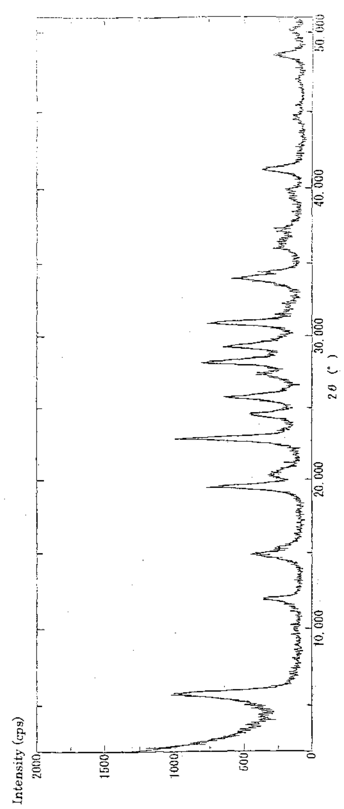


EP 1 508 557 A1

**Description**

TECHNICAL FIELD

The present invention relates to a process for producing halobenzene derivatives that are useful for various organic synthetic chemical substances such as typically medicines and agricultural chemicals and also for polymer materials. More precisely, the invention provides a process for selectively producing para-dihalobenzene derivatives and 1,2,4-substituted trihalobenzene derivatives.

BACKGROUND ART

Halobenzene derivatives are industrially important compounds as starting materials and intermediates for many compounds. Dihalobenzene derivatives include three isomers, ortho, meta and para isomers. In particular, para-halobenzene derivatives are important as starting materials for various organic compounds. Para-dichlorobenzene, a type of para-halobenzene derivatives is has an extremely high industrial value as a starting material for medicines and agricultural chemicals and, in addition, it can be used as insecticide and deodorizer by itself. Trihalobenzene derivatives include three isomers, 1,2,3-trihalobenzene, 1,2,4-trihalobenzene and 1,3,5-trihalobenzene isomers, as classified depending on the positions of the substituents thereof. In particular, 1,2,4-trihalobenzene derivatives that have the smallest molecular size are important as starting materials for various organic compounds. For example, 4-chloro-orthoxylene is useful as a starting material and an intermediate for many useful compounds, for example, as a material for polymers.

Halobenzene derivatives are generally produced by halogenating benzene and/or benzene derivatives with a Lewis acid catalyst such as ferric chloride or aluminium chloride. For example, at present, dichlorobenzene is produced through industrial chlorination that comprises applying a chlorine vapor to benzene or monochlorobenzene in the presence of a Lewis acid catalyst such as ferric chloride or aluminium chloride. However, the ratio of dichlorobenzene isomers produced according to this method is from 30 to 40% of ortho-isomer, about 5% of meta-isomer and from 60 to 70 % of para-isomer, and it is difficult to greatly vary the production ratio of the isomers in the method. Accordingly, many studies have heretofore been made for increasing the para-selectivity in the method. One is to use zeolite as the catalyst. For example, *Journal of Catalysis,* Vol. 60, pp. 110-120 (1979) shows that use of ion-exchangedX-type or Y-type zeolite as the catalyst for liquid-phase bromination of halobenzenes gives para-isomers at a higher selectivity than in conventional methods using a Lewis acid as the catalyst.

JP-A-59-163329 discloses use of L-type zeolite in liquid-phase nucleic halogenation of benzenes to give para-dihalobenzenes at high selectivity.

JP-A-57-77631 shows examples of vapor-phase halogenation, saying that chlorination of monochlorobenzene with a catalyst of zeolite having a pore size of from 5 angstroms to 13 angstroms, for example, Molecular Sieve 5A, 10X or 13X or HY-type zeolite gives paradichlorobenzene at a higher selectivity than in conventional methods.

4-Chloro-orthoxylene is obtained by chlorinating ortho-xylene in the presence of a Lewis acid, but in the process, 3-chloro-orthoxylene and α-chloroxylene that do not so much differ from 4-chloro-orthoxylene in point of the boiling point thereof are produced. For selectively obtaining the useful 4-chloro-orthoxylene, JP-A-3-81234 discloses a method of producing 4-chloro-orthoxylene by chlorinating orthoxylene with a catalyst of L-type zeolite, showing that, when a nitro group-containing compound is added to the reaction system as an additive, then the production ratio of 4-chloro-orthoxylene/3-chloro-orthoxylene increases. In addition, this further says that the chlorinating agent, chlorine may be diluted, in the section of the detailed description of the invention. However, the concentration of the diluted agent is not described therein. In *Journal of Catalysis,* Vol. 150, pp. 430-433 (1994), Sudgit B. Kumaretal. show that when K-L-type zeolite dried at 165°C for 12 hours is used as a catalyst and when 1, 2-dichloroethane is sued as a solvent, then the production ratio of 4-chloro-orthoxylene/3-chloro-orthoxylene increases. JP-B-63-34130 discloses a method for producing 2,5-dichlorotoluene by chlorinating ortho-chlorotoluene with a catalyst of L-type zeolite.

In *Journal of Organic Chemistry,* Vol. 38, No. 6, pp. 1243-1245 (1973), Belnard Mirror discloses a method of selective reductive decomposition of α-halogen in processing three different types of 4-chloro-benzylchloride derivatives with zinc/concentrated hydrochloric acid/ethanol. However, the method is problematic in that a large amount of reagents, 323 % by weight of zinc/1000 % by weight of hydrochloric acid/200 % by weight of ethanol relative to the starting material must be used, and the reaction time and the distillation pre-treatment (extraction, base treatment, drying, filtration) time are long. Forindustrial-scalemassproduction, themethodcouldnotalways be allowed in point of the production costs and the productivity.

In addition, there are many known references that disclose application of a zeolite catalyst as a solid acid catalyst to liquid-phase organic reaction. However, most of these disclose only the catalyst potency of zeolite, and studies of isolation of zeolite catalyst from reaction liquid are not as yet fully advanced.

A zeolite catalyst is generally a crystalline powder. In order that the zeolite catalyst could be effective in a

liquid-phase organic reaction system, the catalyst must be uniformly suspended therein, and, in addition, in the step of separating and recovering the zeolite catalyst after the reaction, it is desirable that the catalyst could rapidly sediment in the reaction liquid and its separability is good. However, when a zeolite catalyst is, as it is, directly suspended and reacted, then it is difficult to separate and recover the catalyst after the reaction. In other words, since the zeolite catalyst poorly sediments in the reaction liquid, the separation of the catalyst from the reaction liquid takes a lot of time, and, in addition, the recovery and handling of the separated catalyst will be troublesome.

If the separation of the zeolite catalyst is insufficient, then it causes some problems in the subsequent step of purification of the reaction product. For example, in distillation purification, the filler in the distillation column maybechokedandtheheatermaybescaled; and in crystallization, the crystals may be contaminated with zeolite powder.

JP-A-2-45431 discloses, as a prior-art technique, a method filtering a zeolite catalyst through a porous ceramic filter that may be intermittently backwashed. JP-A-5-68869 discloses a reaction method of using a cross-flow filter for separation of the particulate solid catalyst from a liquid reaction product obtained in a catalyst suspension fluidized bed reactor. In JP-A-2-45431 and 5-68869, however, the filters are expensive and, in addition, the filter material choked with a zeolite catalyst could not be regenerated in ordinary baking. Further, in continuous reaction lines, a zeolite catalyst must be continuously or intermittently supplied and the used zeolite catalyst must be continuously or intermittently taken out, and the taken-out zeolite catalyst is a slurry. Accordingly, an additional operation is necessary for recovering the zeolite catalyst and the reaction liquid from the slurry, and it is a troublesome operation.

Further, in the cross-flow system in JP-A-5-68869, the flow rate of the processing solution per the cross area of the filter line, or that is, the cross-flow linear speed thereof must be increased for preventing the scaling inside the filter, and the circulation amount through the filter and the reactor shall increase in order to maintain the cross-flow linear speed. When a slurry of zeolite that comprises silica and alumina as the basic structure thereof is passed through the reaction line at a cross-flow linear speed of at most 20 m/sec, then the filter material, the pipe line and the pump may be much worn away and, in addition, the zeolite catalyst may be further ground to give an increased amount of fine particles having a size of 1 $\mu$m or less, and the increased amount of such fine particles could not be ignored. For preventing such fine particles from flowing out through filters to clog them, the filters must have a further smaller pore size and they will be therefore more expensive.

JP-A-63-166434 discloses a method for producing p-DCB (para-dichlorobenzene), which comprises using a shaped zeolite catalyst and in which the separability of the zeolite catalyst from the reaction liquid is improved. However, in the method of JP-A-63-166434, the degree of chlorination is from 98 to 99 % and is low, and much unreacted chloride remains. In addition, the zeolite could not be completely prevented from being broken, though its strength is increased in some degree, and the problems mentioned hereinabove could not be solved as yet.

JP-A-8-40947 discloses a method of separating a zeolite catalyst from the liquid reaction product obtained in a zeolite suspension fluidized bed reactor, in a mode of sedimentation separation of the catalyst.

In such conventional methods, however, the para-selectivity in dihalobenzene derivatives, and the 1,2,4-tri-selectivity in trihalobenzene derivatives are as yet insufficient. In addition, in haloalkylbenzene derivatives, a very small quantity of impurities such as $\alpha$-chloroalkylbenzene derivatives (benzylchloride derivatives) are inevitable as side products. In addition, separation and recovery of catalysts from organic reaction liquids, and recycling of catalysts are unsatisfactory.

Accordingly, in industrial production of dihalobenzene derivatives, it is extremely important to selectively produce para-halobenzene derivatives while the other side products, ortho-halobenzene derivatives and meta-halobenzene derivatives are reduced as much as possible. In producing trihalobenzene derivatives, further increase in the 1,2,4-tri-selectivity is desired. For example, in producing chloro-orthoxylene, when the production of 3-chloro-orthoxylene that does not so much differ from it in point of the boiling point thereof could be reduced and when 4-chloro-orthoxylene of high activity could be produced more selectively at a higher yield, then the purification step in the production process could be simplified and the fixed cost forpurification couldbe reduced. In addition, if $\alpha$-chloro-orthoxylene, which may be in chloro-orthoxylene as an impurity and which could not be readily removed even after distillation owing to the small boiling point difference therebetween, could be rapidly and selectively decomposed without use of a large amount of a catalyst or the like so that high-purity 4-chloro-orthoxylene couldbe efficiently obtained through distillation with no specific treatment thereof, then the load to the step of distillation pretreatment and purification could be reduced and simplified, and the fixed cost of the purification step could be reduced, and the producibility is therefore increased.

In addition, when the catalyst could be recycled, then the proportional cost may be thereby reduced. In the references of conventional methods, the reaction selectivity and the activity of catalysts are disclosed, but nothing is disclosed relating to recycling of zeolite catalysts that are the most expensive of all the materials. Repeatedly recycling catalysts, if possible, may result in reduction in industrial wastes and reduction in production costs.

DISCLOSURE OF THE INVENTION

We, the present inventors have assiduously studied for the purpose of solving the problems as above, and, as a result, have found that, when a zeolite catalyst, which either contains fluorine or has the crystal size of at most 100 nm is used in halogenating benzene or benzene derivatives, then para-substituted halobenzene derivatives or 1,2,4-substituted halobenzene derivatives can be produced at high selectivity and high yield, and have reached the present invention.
To solve the above-mentioned problems, the invention essentially has the following constitution. Specifically, the invention is a process for halogenation of benzene or benzene derivatives, which comprises using a fluorine-containing zeolite catalyst or a zeolite catalyst having the crystal size of at most 100 nm in halogenation of benzene or benzene derivatives.

BRIEF DESCRIPTION OF THE DRAWINGS

Fig. 1 is an X-ray diffraction pattern of the L-type zeolite produced in Example 1.
Fig. 2 is a field-emission scanning electromicroscopic (FE-SEM) image of the L-type zeolite produced in Example 1.
Fig. 3 is a field-emission scanning electromicroscopic (FE-SEM) image of Toso's K-L-type zeolite used in the reaction in Comparative Examples 1 and 2.
Fig. 4 is a particle size distribution pattern of a zeolite catalyst before reaction.
Fig. 5 is a particle size distribution pattern of the zeolite catalyst after reaction.
Fig. 6 shows a flowchart of a process of adding water to a anhydrous liquid-phase organic reaction liquid with a zeolite catalyst suspended therein, then aggregating the zeolite catalyst therein, and separating the zeolite catalyst from the anhydrous liquid-phase organic reaction liquid by the use of a solid-liquid separator.
Fig. 7 shows a flowchart of a process of adding water to a anhydrous liquid-phase organic reaction liquid with a zeolite catalyst suspended therein, until two-phase separation into aqueous phase and organic phase, and then processing the zeolite catalyst-containing aqueous phase for liquid-liquid separation so as to separate the zeolite catalyst from the anhydrous liquid-phase organic reaction liquid.

BEST MODE FOR CARRYING OUT THE INVENTION

The invention is described in detail hereinunder. The process for producing halobenzene derivatives of the invention is characterized in that a zeolite catalyst which either contains fluorine or has the crystal size of at most 100 nm is used in halogenation of benzene or benzene derivatives.
In the process of the invention, a fluorine-containing zeolite catalyst is used. Zeolite as referred to herein is a crystalline microporous material that has a molecular-size, uniform pore diameter, and it includes crystalline aluminosilicate, crystalline metallosilicate, crystalline metalloaluminosilicate, crystalline aluminophosphate, crystalline metalloaluminophosphate, crystalline silicoaluminophosphate. Metallosilicate and metalloaluminosilicate as referred to herein are those derived from aluminosilicate by substituting a part or all of aluminium in aluminosilicate with any other metal than aluminium, such as gallium, iron, titanium, boron, cobalt or chromium. Similarly, metalloaluminophosphate is derived from aluminophosphate by substituting a part of aluminium or phosphorus therein with any other metal.
Zeolite as referred to herein is meant to include all zeolite structures described in *Atlas of Zeolite Structure Types* (W. M. Meier, D. H. Olson, Ch. Baerlocher, Zeolites), 17(1/2), 1996). Any other new-type zeolites not described in this reference are also within the scope of the zeolite for use in the invention. However, preferred for use in the invention are L-type zeolite, faujasite-type zeolite, A-type zeolite, MFI-type zeolite, mordenite-type zeolite, β-type zeolite, Ω-type zeolite, AFI-type zeolite, AEL-type zeolite and ATO-type zeolite that are easily available. More preferred is a zeolite having an alkali metal as the cation thereof. The reason is because the alkali metal in such zeolite could be an active point for chlorine activation. Even more preferred is an L-type zeolite for the reasons that para-dihalobenzenes can be produced at high selectivity, the production ratio of 4-chloro-orthoxylene/3-chloro-orthoxylene increases and the yield of chloro-orthoxylene is high. At present, the reason why the use of L-type zeolite may increase the selectivity is not as yet clarified.
L-type zeolite generally contains K ion as the cation, but it may contain any others than it.
The fluorine-containing zeolite is described. The zeolite contains fluorine, and it means that, when the zeolite is analyzed in a mode of instrumental analysis with an analyzer capable of detecting fluorine and when fluorine is detected in the analyzed zeolite, then the zeolite contains fluorine. The instrumental analysis for fluorine detection includes, for example, atomic absorption analysis, X-ray photoelectric spectrophotometry, X-ray microanalysis, ICP (inductive coupling plasma) analysis, fluorescent X-ray analysis. These methods for analysis are not specifically defined

so far as they enable fluorine detection. Preferably, however, it is desirable that at least 0.05 % by weight of fluorine can be detected in absolute-dry fluorine-containing zeolite. The absolute-dry zeolite is prepared by calcinating zeolite at 500°C for 2 hours and then cooling it in a desiccator filled with diphosphorus pentoxide.

The fluorine-containing zeolite may be obtained by contacting a fluorine-containing compound with zeolite and then calcinating it. The mode of contacting the two is not specifically defined, and it may be effected in a solid phase, in a liquid phase or in a gas phase. Most preferably, a fluorine-containing salt is formed into an aqueous solution thereof, and it is contacted with zeolite. This is preferable since the aqueous solution can be uniformly contacted with zeolite. After thus contacted, the zeolite may be directly dried as it is, or after it is once filtered and then dried without being washed with water. In order that fluorine is finally fixed on zeolite, the temperature in calcinating is not specifically defined. For example, the calcinating may be effected at 300 to 500°C. The fluorine-containing salt is not specifically defined. Its preferred examples are alkali metal salts such as potassium fluoride and sodium fluoride, as well as ammonium fluoride.

Apart from the above, also employable herein is a method of contacting fluorine gas or hydrogen fluoride gas with zeolite.

When the fluorine-containing zeolite catalyst is used, then the selectivity of para-halobenzene derivatives or 1,2,4-substituted halobenzene derivatives increases. At present, however, the reason is not as yet clarified.

Though not specifically defined, the amount of the fluorine-containing zeolite to be used in halogenation may be generally from 0.5 to 60 g per mol of the substrate. In halogenation, when the amount of the zeolite catalyst to be used is smaller, then it is more desirable from the viewpoint of the production costs.

It is important that the zeolite for use in the invention has the crystal size of at most 100 nm. The shape of the crystal is not specifically defined. The size of the crystal may be determined through observation with a scanning electronic microscope. Zeolite is sampled at random, and when the samples are observed with a scanning electronic microscope, then it is desirable that at least 50 % of the crystallites in each sample have a size of at most 100 nm. For accurately judging the crystal size, it is desirable that the samples are observed at amicroscope of 20,000 or more magnifications.

Various methods for producing zeolite have heretofore been disclosed. For example, various production methods are described in *Handbook of Molecular Sieves,* R. Szostak, VAN NOSTRAND RAINHOLD, 1992. In particular, the size of zeolite crystallites varies depending on the reaction mixture composition, the crystallization temperature, the crystallization time and the stirring speed in their production. Therefore, though not indiscriminately defined, the composition ratio in the reaction mixture must be most suitably determined since the size of the crystallite complicatedly varies depending on the concentration of silica and alumina and on the concentration of alkali. Regarding the crystallization condition, when the crystallization temperature is lowered or the crystallization time is shortened, or when the stirring speed is increased, then the size of the crystallites may be reduced. When an organic basic nitrogen compound or a surfactant is in the reaction mixture, then the size of the crystallites may also be reduced.

When the zeolite catalyst having the crystal size of at most 100 nm is used, then the selectivity of para-halobenzene derivatives or 1,2,4-substituted halobenzene derivatives increases. At present, the reason is not as yet clarified.

The amount of the zeolite having the crystal size of at most 100 nm to be used in halogenation is not specificallydefined. In general, it may be from 0.5 to 60 g per mol of the substrate. In halogenation, when the amount of the zeolite catalyst to be used is smaller, then it is more desirable from the viewpoint of the production costs.

In the process of the invention, benzene or benzene derivatives are used as the substrate. The benzene derivatives are meant to indicate compounds that are derived from benzene by substituting the hydrogen atom thereof with a substituent such as halogen or alkyl group, for example, halobenzenes and alkylbenzenes. For example, they are preferably monohalobenzenes, dihalobenzenes, monoalkylbenzenes, or dialkylbenzenes. Especially preferred are chlorobenzene, toluene, orthoxylene. Benzene and benzene derivatives for use herein are not specifically defined in point of their purity, and any ones that are industrially available are usable herein. Their purity is preferably higher, but in general, they contain some impurities and those containing such impurities are applicable to the invention.

Preferably, the water content of the substrate, benzene or benzene derivatives is at most 100 ppm. In the process of the invention, zeolite is used as the catalyst. Zeolite is generally hydrophilic and has the property of extremely selectively absorbing water. Accordingly, when the water content of the substrate is larger than 100 ppm, then the zeolite pores may adsorb water and high selectivity could not be attained. In addition, when water is contained in zeolite, then hydrogen chloride generated in chlorination may dissolve in water adsorbed by the zeolite and it gives hydrochloric acid. As a result, the zeolite structure undergoes aluminium removal, and the catalyst life is thereby shortened. This is undesirable in the invention.

The method of reducing the water content of the substrate is not specifically defined. For example, water-removing molecular sieve may be added to the substrate to remove water; or the substrate may be heated to remove water through evaporation. After water has been thus removed from the substrate, it is important that the substrate is not exposed to moisture-containing air.

The process of the invention is halogenation. The halogenating agent is not specifically defined, and any one may be used herein. For example, it includes fluorine gas, chlorine gas, bromine, iodine, sulfuryl chloride. In chlorination, for example, chlorine gas is most preferred, since it is inexpensive and is easy to handle and since its application is easy to control and its reaction pressure is also easy to control.

In halogenation of the process of the invention, the reaction temperature may be any desired one. However, if the reaction temperature is too high, then side reaction may increase. Therefore, the temperature preferably falls between -50°C and 150°C, more preferably between 0°C and 100°C. Specifically, when the substrate is contacted with a halogenating agent, then the halogenation is attained. In chlorination, for example, chlorine gas is applied to benzene or benzene derivatives. Preferably, the gas is applied to the substrate with stirring. For more efficient utilization of chlorine, it is desirable that chlorine gas is applied to a satisfactory depth of the substrate. The reaction may be effected under increased pressure, atomospheric pressure reduced pressure. For reducing the equipment costs, the reaction is preferably effected under atmospheric pressure.

The reactors for use in the process of the invention may be any of continuous, batch-type or semibatch-type ones.

Preferably, a solvent is added to the halogenation in the invention. For example, in producing 4-chloro-orthoxylene, it is known that, in a conventional method, when a solvent of dichloroethane and a catalyst of K-L-type zeolite are used, then the production ratio of 4-chloro-orthoxylene/3-chloro-orthoxylene increases. We, the present inventors have further tried a fluorine-containing zeolite catalyst or a zeolite catalyst having the crystal size of at most 100 nm, and have found that the selectivity increases beyond expectation, and have reached the present invention. The reason is not as yet clarified.

It is desirable that the solvent used is recovered through distillation and recycled from the viewpoint of the production costs. Accordingly, the solvent for use in the invention preferably has a satisfactory boiling point difference from the product and has a low boiling point. The type of the solvent is not specifically defined. Known halogenated compounds and nitro-containing compounds are preferably used. Halogenated compounds are more desirable than nitro-containing compounds because of higher activity. The halogenated compounds may be aromatic or aliphatic ones. For example, chlorine compounds include chlorobenzene, o-, m-, p-dichlorobenzene, chloroform, carbon tetrachloride, chloroethane, dichloroethane, trichloroethane, chloropropane, dichloropropane. From the viewpoint of the later steps and the recyclability, the compounds having a boiling point of not higher than 160°C are preferred. In particular, dichloroethanes such as 1,2-dichloroethane, and dichloropropanes such as 1,2-dichloropropane and 1,3-dichloropropane are preferred since both the activity and the selectivity are high.

Also preferably, a previously-diluted halogen gas is used in halogenation in the process of the invention. The type of the diluting gas is not specifically defined. In view of the cost and the handlability, inert gas such as nitrogen is most preferred. For example, when such a previously-diluted chlorine gas is used for chlorination, then the selectivity of 1,2,4-tri-substituted products increases in the production of tri-substituted chlorinated derivatives through chlorination of di-substituted benzene derivatives having a substituent at an ortho- or meta-position. For example, in chlorination of orthoxylene, the production ratio of 4-chloro-orthoxylene/3-chloro-orthoxylene increases. In addition, since the production of the side product, polychloro compounds is retarded, the yield of chloro-orthoxylene is further increased. Though the reason is not as yet clarified at present, it may be presumed that, when a diluting gas is mixed with chlorine gas and applied to the substrate, then the side product, hydrochloric acid and others may be removed away from the reaction system owing to the aeration effect of the mixed gas and, as a result, the catalyst surface could be cleaned.

The concentration of the previously-diluted halogen gas is not specifically defined so far at it is lower than 100 mol%. For example, it may be from 5 mol% to 99 mol%, preferably from 5 mol% to 80 mol%, more preferably from 5 mol% to 50 mol%. For example, when the concentration of the chlorine gas is lower than 5 mol% , then the chlorination reaction rate will be extremely lowered and the reaction time will be prolonged. This is rather disadvantageous and unfavorable from the economical viewpoint.

Preferably, L-type zeolite having a water content of at most 1.0% by weight is used for the catalyst.

L-type zeolite is hydrophilic, and when exposed to air, then it adsorbs moisture in air and generally may have a water content of from 7 to 10 %. *Journal of Catalysis,* Vol. 150, pp. 430-433 (1994) discloses a method of activation of catalyst at 165°C for 12 hours. However, even when zeolite is dried according to the method, about 1.4 % by weight of water still remains in the processed zeolite. We, the present inventors have realized the use of an L-type zeolite having a water content of at most 1.0% by weight for the reaction catalyst, according to the method mentioned below, and have found that the L-type zeolite having such a low water content exhibits an extremely high selectivity in a process of producing 4-chloro-orthoxylene, for example, through chlorination of orthoxylene.

(1) L-type zeolite is calcinated at 200°C or higher, preferably at 400°C or higher, and while still hot, this is cooled in a desiccator filled with a drying agent diphosphorus pentoxide, and then this is introduced into orthoxylene in such a manner that the catalyst is prevented as much as possible from being exposed to moisture-containing air.
(2) L-type zeolite having a water content of larger than 1 % is introduced into orthoxylene and heated therein at

100°C or higher so as to be dried, and the resulting zeolite is directly used in reaction, not exposed to air.
(3) L-type zeolite is introduced into a reactor and, while dry gas is introduced thereinto, this is heated at 100°C to be dried, and, without exposing the resulting zeolite to moisture-containing air, orthoxylene is introduced into it and the reaction is started.

A method of measuring the water content of L-type zeolite is described below. The weight of zeolite is measured in such a manner that it is not exposed to air as much as possible, and this is W1. The zeolite is put into an electric furnace at 500°C and calcinated therein for 3 hours, and while still hot, this is put into a desiccator filled with diphosphorus pentoxide and cooled therein. After this is cooled to room temperature, its weight is measured in such a manner that it is not exposed to air as much as possible, and this is W2. The water content of the zeolite is 100 × (W1 - W2) /W1 (%).
In the process of producing halobenzene derivatives of the invention, which comprises halogenating benzene derivatives, it is important that after the benzene derivative has been 100% converted, it is further halogenated. Heretofore, it has been said that, if the conversion of benzene derivatives is too high, then halobenzene derivatives are further halogenated into dihalobenzene derivatives and this is unfavorable since the unit requirement of benzene derivatives is lowered. However, we, the present inventors have found that, when a benzene derivative has been 100% converted and it is further halogenated, then the selectivity of para-substituted halobenzene derivatives or 1,2,4-substituted halobenzene derivatives is greatly increased relative to ortho-substituted halobenzene derivatives or 1,2,3-substituted halobenzene derivatives or α-halobenzenes, since ortho-substituted halobenzene derivatives or 1,2,3-substituted halobenzene derivatives are more readily dichlorinated to give high-boiling-point compounds than para-substituted halobenzene derivatives or 1,2,4-substituted halobenzene derivatives. The unit requirement of benzene derivatives may surely lower, but the production of ortho-substituted halobenzene derivatives or 1,2,3-substituted halobenzene derivatives as well as α-halobenzenes that are difficult to separate is reduced and the load to the complicated purification step after the reaction may be reduced. Concretely, the product could be easily purified to a high degree even in a distillation column having a reduced number of stages, and therefore the yield in the purification step increases. As a whole, therefore, halobenzene derivatives can be produced at high yield and at low costs. Further halogenation after 100 % conversion of benzene derivatives may be attained by prolonging the reaction time for halogenation.
In particular, in a process of obtaining 4-chloro-orthoxylene by chlorinating orthoxylene, it is important that orthoxylene is converted to a degree of 95% or more. Heretofore, it has been said that, if the conversion of orthoxylene is too high, then 4-chloro-orthoxylene is further chlorinated into dichloro-orthoxylene and this is unfavorable since the unit requirement of orthoxylene is lowered. However, we, the present inventors have found that, when the conversion of orthoxylene is over 95%, then the selectivity of 4-chloro-orthoxylene is greatly increased relative to 3-chloro-orthoxylene and α-chloro-orthoxylene, since 3-chloro-orthoxylene and α-chloro-orthoxylene are more readily dichlorinated to give high-boiling-point compounds than 4-chloro-orthoxylene. The unit requirement of orthoxylene may surely lower, but the production of 3-chloro-orthoxylene and α-chloro-orthoxylene that are difficult to separate is reduced and the load to the complicated purification step after the reaction may be reduced. Concretely, the product could be easily purified to a high degree even in a distillation column having a reduced number of stages, and therefore the yield in the purification step increases. As a whole, therefore, 4-chloro-orthoxylene can be produced at high yield and at low costs. For increasing the conversion of orthoxylene to 95 % or more, the reaction time is prolonged until the conversion reaches at least 95 %. The conversion of 100 % or more is more preferable since the selectivity is further increased.
In the process of the invention, it is desirable that from 0.001 to 10 % by weight of a metal and/or a metal salt is added to a mixture that comprises a haloalkylbenzene derivative (I) of the following general formula (I) and contains at least one impurity of an α-haloalkylbenzene derivative (II) of the following general formula (II) so as to selectively decompose the α-haloalkylbenzene derivative (II), and the mixture is preferably purified through distillation into a high-purity nucleus-halogenated alkylbenzene derivative.

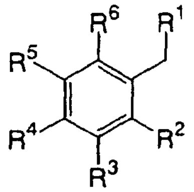

(I)

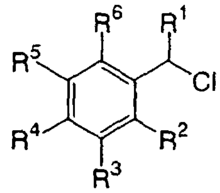

(II)

(In formula (I), the substituents $R^1$ to $R^6$ are independent of each other; $R^1$ represents a hydrogen atom, or an alkyl group having from 1 to 4 carbon atoms; $R^2$ to $R^6$ each represent a hydrogen atom, a halogen atom, or an alkyl group having from 1 to 5 carbon atoms, and at least one or more of these substituents are any of a chlorine atom and an alkyl group having from 1 to 5 carbon atoms. In formula (II), the substituents $R^1$ to $R^6$ are independent of each other; $R^1$ represents a hydrogen atom, or an alkyl group having from 1 to 4 carbon atoms; $R^2$ to $R^6$ each represent a hydrogen atom, a halogen atom, an alkyl group having from 1 to 5 carbon atoms, or an $\alpha$-haloalkyl group having from 1 to 5 carbon atoms, and at least one or more of these substituents are any of a chlorine atom and a hydrogen atom.)

Compounds of formulae (I) and (II) are described. In formula (I), the groups $R^1$ to $R^6$ are independent of each other, and when $R^1$ is a hydrogen atom, $R^2$ to $R^6$ each are a hydrogen atom, a chlorine atom or a methyl group, and one of these groups is a chlorine atom or a methyl group; and in formula (II), the substituents $R^1$ to $R^6$ are independent of each other, and when $R^1$ is a hydrogen atom, $R^2$ to $R^6$ each are a hydrogen atom, a chlorine atom, a methyl group or an $\alpha$-chloromethyl group, and at least one of these group is a chlorine atom, a hydrogen atom or a methyl group, then the invention produces good results.

The impurities in the mixture include one or more types of $\alpha$-haloalkylbenzene derivatives (II), regio-isomers of haloalkylbenzene derivatives, compound groups produced through excess chlorination of the desired haloalkylbenzene derivatives, and residual solvents, but the impurities are not limited to these.

It is believed that the selective decomposition of $\alpha$-haloalkylbenzene derivatives in the invention may include Friedel-Crafts type alkylation. The minor amount of metal for selective decomposition of $\alpha$-haloalkylbenzene derivatives may be at least one selected from iron, aluminium, antimony, gallium, zirconium, tin, boron and their salts. The salts are preferably $FeCl_3$, $AlCl_3$, $AlBr_3$, $SbCl_5$, $GaCl_3$, $ZrCl_4$, $BCl_3$, and $BF_3$. More preferred are $FeCl_3$, $AlCl_3$, and Fe in view of the rapid reaction rate and the cost of the metals. Needless-to-say, these are not limitative. The amount of the metal to be added to the system is at most 10 % by weight of the mixture comprising, as the essential ingredient thereof, haloalkylbenzene derivatives that are to be purified, and the selective decomposition may be finished within a few minutes. In view of the producibility and the production costs, the metal amount is preferably from 0.001 to 10% by weight, more preferably from 0.01 to 5 % by weight.

Regarding its temperature, the decomposition may be attained at any reaction temperature not lower than room temperature. However, if the reaction temperature is low, then the reaction rate will be low. Preferably, the reaction temperature falls between room temperature and 200°C, more preferably between 60°C and 200°C. The reaction may go on in the absence of a solvent, but when the content of the impurity, $\alpha$-haloalkylbenzene is over 5 %, the yield may reduce. In such a case, therefore, it is desirable that an aromatic solvent such asbenzene, toluene orxylene is added to the system in an equimolar amount thereof relative to $\alpha$-haloalkylbenzene. If desired, for example, dichloropropane, ethylene dichloride, methylene chloride, chloroform, hydrochloric acid/ethanol, or hydrochloric acid/methanol solvent may also be used. Except the case where hydrochloric acid/ethanol or hydrochloric acid/methanol is used as the solvent, the water content of the mixture to be decomposed is preferably as small as possible. More preferably, it is at most 1000 ppm, even more preferably at most 100 ppm. However, even when the water content is larger than it, the intended decomposition may be attained so far as excess metal and/ormetal salt is added to the system. Therefore, the water content of the mixture is not specifically defined. The decomposition reaction time may be within 10 minutes so far as the decomposition temperature, and the type and the amount of the metal used are suitably determined. After the decomposition, any additional treatment for metal removal is unnecessary, and the distillation purification may be finished with no additional treatment thereto.

The decomposition reaction may be effected under increased pressure, atmospheric pressure or reduced pressure, but is preferably effected under atmospheric pressure from the viewpoint of reducing the equipment costs.

The decomposition reactor for use in the process of the invention may be any of continuous, batch-type or semibatch-type ones.

After the reaction according to the process of the invention, zeolite is separated from the zeolite-containing, anhydrous liquid-phase organic reaction liquid. For the separation, it is desirable that water is added to the anhydrous liquid-phase organic reaction liquid to aggregate the dispersed zeolite and then this is introduced into a solid-liquid separator to separate the zeolite catalyst therein.

In the method of separating the zeolite catalyst in the invention, we, the present inventor do not intend at all to define a preferred range of the mean particle size of the zeolite.

In this connection, even when an unused zeolite catalyst has a sufficiently large particle size and, after used, it could be readily separated and recovered in any known separation method of sedimentation separation, the zeolite catalyst may be ground and broken during reaction while stirred in a reactor or depending on the reaction condition such as the slurry concentration. For example, a commercially-available zeolite catalyst is formulated to have a slurry concentration of 2% by weight and this is subjected to anhydrous organic reaction for 30 hours. Fig. 4 and Fig. 5 show a particle size distribution pattern of the zeolite catalyst before and after the reaction. Trough the reaction, about 10 % of the aggregated zeolite particles were ground and broken owing to the stirring in the reactor and to the collision of the zeolite catalyst particles to each other. The present invention may apply even to separation and recovery of zeolite catalysts of the type that are ground and broken and are could therefore poorly sediment in anhydrous organic reaction liquids.

The content of the zeolite catalyst in the anhydrous organic reaction liquid to be processed is not specifically defined. Preferably, it is at most 35% by weight of the overall amount of the anhydrous organic reaction liquid. In general, when the content of the zeolite catalyst is over 35 % by weight, then the slurry flowability is lowered and the operability is therefore lowered.

Regarding the addition of water to the reaction system, water may be directly supplied to the reactor after the reaction. Preferably, however, water is added to a branch line which is connected to the reactor and to which the anhydrous organic reaction liquid is led from the reactor, and this is stirred with a line mixer or the like. More preferably, a tank for water addition and mixture may be provided after the reactor.

When a small amount of water (from 0.001 to 1.0 time by weight of the zeolite catalyst weight in the anhydrous liquid-phase organic reaction liquid) is added so as to aggregate the zeolite catalyst in the anhydrous liquid-phase organic reaction liquid, the resulting mixture may be directly filtered after stirred and mixed. Preferably, however, the mixture is filtered via a tank in which the crystal may be fully aggregated therein. The amount of water to be added may be from 0.001 to 1.0 time by weight of the zeolite catalyst weight in the anhydrous organic reaction liquid. Preferably, it is at most 0.2 times by weight. Though depending on the solubility in water of the anhydrous liquid-phase organic reaction liquid, when water is added in an amount of from 1.0 to 1.5 times by weight of the zeolite catalyst, then the aggregated particles of the zeolite catalyst may be clayish and may stick to the walls of the reactor and, as a result, their flowability and operability will be poor. So far as the amount of water added is adequate, then the zeolite catalyst may form good aggregated particles and its operability for separation is good.

On the other hand, when water is added until the system is separated into an organic phase and an aqueous phase (the amount of water added is from 1.8 to 100 times by weight of the zeolite catalyst weight in the anhydrous liquid-phase organic reaction liquid) , then the mixture is, after stirred and mixed, led into a tank for liquid-liquid separation equipped with a surface level meter or the like, and the zeolite catalyst having moved to the aqueous phase is separated from the anhydrous liquid-phase organic reaction liquid alongwith the aqueous phase from it. Preferably, the amount of water to be added is so controlled that the slurry concentration of the zeolite catalyst could be at most 35 % by weight, in view of the slurry flowability and operability of the aqueous phase into which the zeolite catalyst has moved.

The slurry concentration is defined by the following formula:

$$\text{Slurry Concentration (wt.\%) of Aqueous Phase} = [(\text{zeolite catalyst weight in anhydrous liquid-phase organic reaction liquid})/(\text{zeolite catalyst weight in anhydrous liquid-phase organic reaction liquid} + \text{added water weight})] \times 100.$$

For example, when the slurry concentration of the zeolite catalyst in a anhydrous liquid-phase organic reaction liquid is 1 % by weight, then water is added in an amount of 1.86 times by weight of the zeolite catalyst weight in order that the slurry concentration of the aqueous phase into which the zeolite catalyst has moved could be at most 35 %.

More preferably, the amount of water to be added is from 2 to 5 times by weight of the zeolite catalyst weight.

The uppermost limit of the amount of water to be added is not specifically defined. However, if the slurry concentration of the zeolite catalyst in the aqueous phase is too much lowered in industrial-scale lines, then it is unfavorable since the line scale and the load to waste water treatment may increase.

The invention is described with reference to the drawings.

Fig. 6 is a flowchart of one example of the process of the invention, in which a small amount of water is added to the system so as to aggregate the zeolite catalyst suspended in a anhydrous liquid-phase organic reaction liquid, and then this is subjected to solid-liquid separation. The anhydrous organic reaction liquid (1) is taken out from a reactor, and its flow rate is controlled by the flow meter (2) equipped with a sensor and by the flow rate-controlling valve (3), and water is added thereto via the flow meter (4) equipped with a sensor and the valve (5) that act for water flow rate control to a predetermined ratio relative to the anhydrous organic reaction liquid flow rate output data from the sensor thereof in such a controlled manner that a predetermined amount of water could be added to the line. After the addition, this is stirred and mixed by the line mixer (6), and then led to the solid-liquid separator (7).

For the solid-liquid separator (7), employable are filters, centrifuges and thickeners, but filters are preferably selected. For use in the process, a pressure-backwashable, closed-type filter is especially preferred, which has a cylindrical filter structure of a filter cloth or a ceramic filter installed inside it.

A filter cloth is used for the filter structure for the solid-liquid separator in Fig. 6. The filter of the type is as follows: The mixture to be filtered is passed through it, and the zeolite catalyst having stuck to the filter cloth is dried through aeration, and peeled from it in a mode of back-blowing of an inert gas such as pressure nitrogen thereto, and the thus-solidified zeolite catalyst is taken out from the bottom of the filter (8). For example, the filter of the type is a FUNDABAC filter (IHI FUNDABAC filter). The solid zeolite catalyst thus collected is dried, subjected to solvent removal, and calcinated, and it may be recycled as a regenerated zeolite catalyst.

On the other hand, the anhydrous liquid-phase organic reaction liquid from which the zeolite catalyst has been removed is taken out of the solid-liquid separator as a filtrate (9), and this is then led to the next purification step.

When the anhydrous liquid-phase organic reaction liquid contains a material to be recycled, or that is, when the anhydrous organic reaction liquid contains a reaction solvent or an unreacted starting material and when this is recovered and recycled to the reactor, then water may dissolve in the anhydrous liquid-phase organic reaction liquid to a degree lower than the solubility thereof since water is added to the system in this process. For example, when the component to be recycled is a light-boiling-point substance relative to the product produced through anhydrous liquid-phase organic reaction, then the component that contains water and is to be recycled is taken out from the distillate (11) from the distillation column (10) where the light-boiling-point substance is separated, and when the distillate condensate could undergo two-phase separation, then a water separator tank is provided in the line and the organic phase (12) separated in the tank may be directly recycled to the reactor. When water may lower the zeolite catalyst potency, then the water-containing catalyst is passed through the water removal column (13), or it is dried in a drying column provided in the line, and then it may be recycled.

Water (14) having been removed from the catalyst may be processed for drainage, but is preferably reused for water to be added to the anhydrous liquid-phase organic reaction liquid for separation of the zeolite catalyst from it.

Fig. 7 is a flowchart of one example of the process of the invention, in which excess water is added to a anhydrous liquid-phase organic reaction liquid until two-phase separation into aqueous phase and organic phase, and the zeolite catalyst having moved to the aqueous phase is separated through liquid-liquid separation from the anhydrous liquid-phase organic reaction liquid along with the aqueous phase from it. The process is effective when the slurry concentration of the zeolite catalyst in the anhydrous liquid-phase organic reaction liquid is low and when the scale of the step of separation and recovery of the zeolite catalyst is great. In such a case, the zeolite catalyst can be efficiently concentrated in the aqueous phase according to the process of this type, and the scale of the step of separation and recovery of the zeolite catalyst may be reduced.

The anhydrous organic reaction liquid (1) is taken out from a reactor, and its flow rate is controlled by the flow meter (2) equipped with a sensor and by the flow rate-controlling valve (3), and water is added thereto via the flow meter (4) and the valve (5) that act for water flow rate control to a predetermined ratio relative to the anhydrous organic reaction liquid flow rate output data from the sensor thereof in such a controlled manner that a predetermined amount of water could be added to the line.

After water is added to the anhydrous organic reaction liquid, the two are mixed by the line mixer (6) and led to the liquid separation tank (14). The liquid separation tank is a liquid-liquid separable tank equipped with a surface level meter or the like. When the organic phase is light, then the anhydrous liquid-phase organic reaction liquid is taken out from the top of the column (15) and the aqueous phase that contains the zeolite catalyst is taken out from the bottom thereof (16). The organic phase of the anhydrous organic reaction liquid is processed in the same manner as in the process of Fig. 6. The zeolite catalyst-containing aqueous phase is led to a solid-liquid separator like that in Fig. 6, or is led to a decanter in which the grown zeolite particles are recovered. The filtrate contains fine particles of the zeolite catalyst, and it is recycled for water to be added to the liquid-phase organic reaction liquid.

EXAMPLES

The invention is described with reference to the following Examples.

(Example 1) Preparation of fluorine-containing L-type zeolite:

6.43 g of anhydrous potassium fluoride and 60 ml of distilled water were added to 10 g (absolute dry) of Tosoh's K-L-type zeolite, stirred (75°C) for 5 hours, and filtered, and the resulting cake was dried overnight at 120°C.

(Example 2) Preparation of fluorine-containing L-type zeolite:

3.22 g of anhydrous potassium fluoride and 60 ml of distilled water were added to 10 g (absolute dry) of Tosoh's K-L-type zeolite, stirred (room temperature) for 10 minutes, and filtered, and the resulting cake was dried overnight at 120°C.

(Example 3) Chlorination with a catalyst of fluorine-containing L-type zeolite:

A 100-ml four-neck flask was equipped with a reflux condenser, a thermometer, a chlorine inlet duct, and a sampling duct. This was so designed that waste chlorine gas could be exhausted from the top of the reflux condenser and trapped in an aqueous sodium hydroxide solution. Heating it was effected in an oil bath.

16.96 g of orthoxylene (Sigma Aldrich Japan' s first class grade chemical) that had been previously dried with Molecular Sieve 4A to have a water content of 20 ppm, 60 ml of 1,2-dichloroethane (Sigma Aldrich Japan's first class grade chemical), and 3.39g of the fluorine-containingK-L-type zeolite (from Tosoh) that had been prepared in Example 1 and calcinated at 400°C for 1 hour and then cooled in a desiccator were put into the flask, and heated at 80°C with nitrogen gas being introduced thereinto through the chlorine gas inlet tube. After this was heated at 80°C, the nitrogen gas was exchanged for chlorine gas and the reaction was started. The chlorine gas flow rate was controlled to be 0.08 mol/hr. The water content of the fluorine-containing K-L-type zeolite that had been prepared in Example 1 and calcinated at 400°C for 1 hour was 0.1% by weight.

After 3 hours, the orthoxylene conversion was 64%, and the production ratio of 4-chloro-orthoxylene/3-chloro-orthoxylene was 9.8.

(Comparative Example 1)

A 100-ml four-neck flask was equipped with a reflux condenser, a thermometer, a chlorine inlet duct, and a sampling duct. This was so designed that waste chlorine gas could be exhausted from the top of the reflux condenser and trapped in an aqueous sodium hydroxide solution. Heating it was effected in an oil bath.

16.96 g of orthoxylene (Sigma Aldrich Japan's first class grade chemical) that had been previously dried with Molecular Sieve 4A to have a water content of 20 ppm, 60 ml of 1,2-dichloroethane (Sigma Aldrich Japan's first class grade chemical), and 3.39 g of a K-L-type zeolite (from Tosoh) that had been calcinated at 400°C for 1 hour and then cooled in a desiccator were put into the flask, and heated at 80°C with nitrogen gas being introduced thereinto through the chlorine gas inlet tube. After this was heated at 80°C, the nitrogen gas was exchanged for chlorine gas and the reaction was started. The chlorine gas flow rate was controlled to be 0.08 mol/hr. The water content of the K-L-type zeolite that had been calcinated at 400°C for 1 hour was 0.1 % by weight.

After 2 hours, the orthoxylene conversion was 49%, and the production ratio of 4-chloro-orthoxylene/3-chloro-orthoxylene was 8.1. After 4 hours, the orthoxylene conversion was 94%, and the production ratio of 4-chloro-orthoxylene/3-chloro-orthoxylene was 8.5.

(Example 4) Chlorination with a catalyst of fluorine-containing L-type zeolite:

A 100-ml four-neck flask was equipped with a reflux condenser, a thermometer, a chlorine inlet duct, and a sampling duct. This was so designed that waste chlorine gas could be exhausted from the top of the reflux condenser and trapped in an aqueous sodium hydroxide solution. Heating it was effected in an oil bath.

53 g of orthoxylene (Sigma Aldrich Japan's first class grade chemical) that had been previously dried with Molecular Sieve 4A to have a water content of 20 ppm, and 2.5 g of the fluorine-containing K-L-type zeolite (from Tosoh) that had been prepared in Example 1 and calcinated at 400°C for 1 hour and then cooled in a desiccator were put into the flask, and heated at 80°C with nitrogen gas being introduced thereinto through the chlorine gas inlet tube. After this was heated at 80°C, the nitrogen gas was exchanged for chlorine gas and the reaction was started. The chlorine gas flow rate was controlled to be 0.13 mol/hr. The water content of the fluorine-containing K-L-type zeolite

that had been prepared in Example 1 and calcinated at 400°C for 1 hour was 0.1% by weight.

After 4 hours, the orthoxylene conversion was 42%, and the production ratio of 4-chloro-orthoxylene/3-chloro-orthoxylene was 1.8.

(Comparative Example 2)

A 100-ml four-neck flask was equipped with a reflux condenser, a thermometer, a chlorine inlet duct, and a sampling duct. This was so designed that waste chlorine gas could be exhausted from the top of the reflux condenser and trapped in an aqueous sodium hydroxide solution. Heating it was effected in an oil bath.

53 g of orthoxylene (Sigma Aldrich Japan's first class grade chemical) that had been previously dried with Molecular Sieve 4A to have a water content of 20 ppm, and 2.5 g of a K-L-type zeolite (from Tosoh) that had been calcinated at 400°C for 1 hour and then cooled in a desiccator were put into the flask, and heated at 80°C with nitrogen gas being introduced thereinto through the chlorine gas inlet tube. After this was heated at 80°C, the nitrogen gas was exchanged for chlorine gas and the reaction was started. The chlorine gas flow rate was controlled to be 0.13 mol/hr. The water content of the K-L-type zeolite that had been calcinated at 400°C for 1 hour was 0.1% by weight.

After 4 hours, the orthoxylene conversion was 46 %, and the production ratio of 4-chloro-orthoxylene/3-chloro-orthoxylene was 1.6.

(Example 5) Chlorination with a catalyst of fluorine-containing L-type zeolite:

A 100-ml four-neck flask was equipped with a reflux condenser, a thermometer, a chlorine inlet duct, and a sampling duct. This was so designed that waste chlorine gas could be exhausted from the top of the reflux condenser and trapped in an aqueous sodium hydroxide solution. Heating it was effected in an oil bath.

16.96 g of orthoxylene (Sigma Aldrich Japan's first class grade chemical) that had been previously dried with Molecular Sieve 4A to have a water content of 20 ppm, 60 ml of 1,2-dichloroethane (Sigma Aldrich Japan's first class grade chemical), and 0.17 g of the fluorine-containing K-L-type zeolite (from Tosoh) that had been prepared in Example 2 and calcinated at 400°C for 1 hour and then cooled in a desiccator were put into the flask, and heated at 80°C with nitrogen gas being introduced thereinto through the chlorine gas inlet tube. After this was heated at 80°C, the nitrogen gas was exchanged for chlorine gas and the reaction was started. The chlorine gas flow rate was controlled to be 0.026 mol/hr. The water content of the fluorine-containing K-L-type zeolite that had been prepared in Example 2 and calcinated at 400°C for 1 hour was 0.1 % by weight.

After 6 hours, the orthoxylene conversion was 97 %, and the production ratio of 4-chloro-orthoxylene/3-chloro-orthoxylene was 8.7.

(Example 6) Chlorination with a catalyst of fluorine-containing L-type zeolite:

A 100-ml four-neck flask was equipped with a reflux condenser, a thermometer, a chlorine inlet duct. and a sampling duct. This was so designed that waste chlorine gas could be exhausted from the top of the reflux condenser and trapped in an aqueous sodium hydroxide solution. Heating it was effected in an oil bath.

16.96 g of orthoxylene (Sigma Aldrich Japan's first class grade chemical) that had been previously dried with Molecular Sieve 4A to have a water content of 20 ppm, 60 ml of 1,2-dichloroethane (Sigma Aldrich Japan's first class grade chemical), and 0.17 g of the fluorine-containing K-L-type zeolite (from Tosoh) that had been prepared in Example 2 and calcinated at 400°C for 1 hour and then cooled in a desiccator were put into the flask, and heated at 80°C with nitrogen gas being introduced thereinto through the chlorine gas inlet tube. After this was heated at 80°C, the nitrogen gas was mixed with chlorine gas and the reaction was started. The gas blend ratio was nitrogen gas/chlorine gas = 0.05 by volume. The chlorine gas flow rate was controlled to be 0.026 mol/hr. The water content of the fluorine-containing K-L-type zeolite that had been prepared in Example 2 and calcinated at 400°C for 1 hour was 0.1 % by weight.

After 6 hours, the orthoxylene conversion was 98 %, and the production ratio of 4-chloro-orthoxylene/3-chloro-orthoxylene was 8.8.

(Example 7) Chlorination with a catalyst of fluorine-containing L-type zeolite:

The same process as in Example 6 was carried out, in which, however, the gas blend ratio was nitrogen gas/chlorine gas = 0.25 by volume.

After 6 hours, the orthoxylene conversion was 96%, and the production ratio of 4-chloro-orthoxylene/3-chloro-orthoxylene was 8.9.

(Example 8) Chlorination with a catalyst of fluorine-containing L-type zeolite:

The same process as in Example 6 was carried out, in which, however, the gas blend ratio was nitrogen gas/chlorine gas = 1 by volume.

After 6 hours, the orthoxylene conversion was 97%, and the production ratio of 4-chloro-orthoxylene/3-chloro-orthoxylene was 9.2.

(Example 9) Chlorination with a catalyst of fluorine-containing L-type zeolite:

The same process as in Example 6 was carried out, in which, however, the gas blend ratio was nitrogen gas/chlorine gas = 2 by volume.

After 6 hours, the orthoxylene conversion was 97%, and the production ratio of 4-chloro-orthoxylene/3-chloro-orthoxylene was 9.9.

(Example 10) Chlorination with a catalyst of fluorine-containing L-type zeolite:

The same process as in Example 6 was carried out, in which, however, the gas blend ratio was nitrogen gas/chlorine gas = 4 by volume.

After 6 hours, the orthoxylene conversion was 94%, and the production ratio of 4-chloro-orthoxylene/3-chloro-orthoxylene was 10.3.

(Example 11) Chlorination with a catalyst of fluorine-containing L-type zeolite:

The same process as in Example 6 was carried out, in which, however, the gas blend ratio was nitrogen gas/chlorine gas = 9 by volume.

After 6 hours, the orthoxylene conversion was 85 %, and the production ratio of 4-chloro-orthoxylene/3-chloro-orthoxylene was 10.4.

(Example 12) Chlorination with a catalyst of fluorine-containing L-type zeolite:

The same process as in Example 6 was carried out, in which, however, the gas blend ratio was nitrogen gas/chlorine gas = 30 by volume.

After 6 hours, the orthoxylene conversion was 70 %, and the production ratio of 4-chloro-orthoxylene/3-chloro-orthoxylene was 10.5.

(Comparative Example 3)

A 100-ml four-neck flask was equipped with a reflux condenser, a thermometer, a chlorine inlet tube, and a sampling duct. This was so designed that waste chlorine gas could be exhausted from the top of the reflux condenser and trapped in an aqueous sodium hydroxide solution. Heating it was effected in an oil bath.

16. 96 g of orthoxylene (Sigma Aldrich Japan' s first class grade chemical) that had been previously dried with Molecular Sieve 4A to have a water content of 20 ppm, 60 ml of 1,2-dichloroethane (Sigma Aldrich Japan's first class grade chemical), and 0.17 g of a K-L-type zeolite (from Tosoh) that had been calcinated at 400°C for 1 hour and then cooled in a desiccator were put into the flask, and heated at 80°C with nitrogen gas being introduced thereinto through the chlorine gas inlet tube. After this was heated at 80°C, the nitrogen gas was mixed with chlorine gas and the reaction was started. The gas blend ratio was nitrogen gas/chlorine gas = 4 by volume. The chlorine gas flow rate was controlled to be 0.026 mol/hr. The water content of the K-L-type zeolite that had been calcinated at 400°C for 1 hour was 0.1% by weight.

After 6 hours, the orthoxylene conversion was 91%, and the production ratio of 4-chloro-orthoxylene/3-chloro-orthoxylene was 7.5.

(Example 13) Production of L-type zeolite having the crystal size of at most 100 nm:

Using Ludox HS-30 (from DuPont) as a silica source, aluminium hydroxide (from Wako Pure Chemicals) as an aluminium source, and potassium hydroxide (having a KOH content of 85.5 wt.% and an $H_2O$ content of 14.5 wt.%, from Sigma Aldrich Japan) as a potassium source, a mixture of the following composition was prepared.

$8K_2O:Al_2O_3:20SiO_2:200H_2O$ (by mol)

Concretely, 62. 99 g of potassium hydroxide was dissolved in 35.35 g of distilled water, and then 9.36 g of

aluminium hydroxide was added thereto and stirred for 30 minutes to give a uniform solution. 240.4 g of Ludox HS-30 was gradually added to the resulting mixture with stirring, and this was stirred for further 3 hours to prepare a uniform, slurry-like aqueous reaction mixture. The reaction mixture was put in a 500-ml autoclave and sealed up, and then reacted at 150°C for 5 days with stirring at 150 rpm.

After the reaction, this was washed with distilled water and then filtered, and this operation was repeated three times. Then, this was dried overnight at about 120°C. The resulting product was analyzed through X-ray diffraction, and its pattern is as in Fig. 1. This confirms that the product is an L-type zeolite. The X-ray diffraction condition was as follows: The X-ray source was Cu/40 kV/30 mA, and the scanning speed was 3 degrees/min. The product was analyzed for its morphology with a field emission scanning electronic microscope (FE-SEM), and Fig. 2 shows its image. This confirms that almost all the crystallites had a size of at most 100 nm.

(Comparative Example 4)

Tosoh's K-L-type zeolite was analyzed for its morphology with a field emission scanning electronic microscope (FE-SEM), and Fig. 3 shows its image. This confirms that almost all the crystals had a size of from 300 nm to 500 nm.

(Example 14) Production of L-type zeolite having the crystal size of at most 100 nm:

Using hydrous silicate (having an $SiO_2$ content of 91.6 wt.%, an $Al_2O_3$ content of 0.33 wt.%, an NaOH content of 0.27 wt.%, and an $H_2O$ content of 7.8 wt.%, Nipsil VN-3 from Nippon Silica) as a silica source, aluminium hydroxide (from Wako Pure Chemicals) as an aluminium source, and potassium hydroxide (having a KOH content of 85.5 wt.% and an $H_2O$ content of 14.5 wt. %, from Sigma Aldrich Japan) as a potassium source, a mixture of the following composition was prepared.

$8K_2O:Al_2O_3:20SiO_2:400H_2O$ (by mol)

Concretely, 94.48 g of potassium hydroxide was dissolved in 50.0 g of distilled water, and then 13.45 g of aluminium hydroxide was added thereto and stirred for 30 minutes to give a uniform solution. 570.4 g of distilled water and 118.14 g of hydrous silicate were gradually added to the resulting mixture with stirring, and this was stirred for further 3 hours to prepare a uniform, slurry-like aqueous reaction mixture. The reaction mixture was put in a 1000-ml autoclave and sealed up, and then reacted at 150°C for 4 days with stirring at 150 rpm.

After the reaction, this was washed with distilled water and then filtered, and this operation was repeated three times. Then, this was dried overnight at about 120°C. The resulting product was an L-type zeolite, in which almost all the crystals had a size of at most 100 nm.

(Example 15) Chlorination with a catalyst of L-type zeolite having the crystal size of at most 100 nm:

A 100-ml four-neck flask was equipped with a reflux condenser, a thermometer, a chlorine inlet tube, and a sampling duct. This was so designed that waste chlorine gas could be exhausted from the top of the reflux condenser and trapped in an aqueous sodium hydroxide solution. Heating it was effected in an oil bath.

16.96 g of orthoxylene (Sigma Aldrich Japan's first class grade chemical) that had been previously dried with Molecular Sieve 4A to have a water content of 20 ppm, 60 ml of 1,2-dichloroethane (Sigma Aldrich Japan's first class grade chemical), and 3.39 g of the L-type zeolite having the crystal size of at most 100 nm that had been prepared in Example 13 and calcinated at 400°C for 1 hour and then cooled in a desiccator were put into the flask, and heated at 80°C with nitrogen gas being introduced thereinto through the chlorine gas inlet duct. After this was heated at 80°C, the nitrogen gas was exchanged for chlorine gas and the reaction was started. The chlorine gas flow rate was controlled to be 0.08 mol/hr. The water content of the L-type zeolite having the crystal size of at most 100 nm that had been prepared in Example 13 and calcinated at 400°C for 1 hour was 0.1% by weight.

After 2 hours, the orthoxylene conversion was 58%, and the production ratio of 4-chloro-orthoxylene/3-chloro-orthoxylene was 8.9.

(Example 16) Chlorination with a catalyst of L-type zeolite having the crystal size of at most 100 nm:

A 100-ml four-neck flask was equipped with a reflux condenser, a thermometer, a chlorine inlet tube, and a sampling duct. This was so designed that waste chlorine gas could be exhausted from the top of the reflux condenser and trapped in an aqueous sodium hydroxide solution. Heating it was effected in an oil bath.

53 g of orthoxylene (Sigma Aldrich Japan's first class grade chemical) that had been previously dried with Molecular Sieve 4A to have a water content of 20 ppm, and 2.5 g of the L-type zeolite having the crystal size of at most 100 nm that had been prepared in Example 13 and calcinated at 400°C for 1 hour and then cooled in a desiccator were put into the flask, and heated at 100°C with nitrogen gas being introduced thereinto through the chlorine gas inlet

tube. After this was heated at 100°C, the nitrogen gas was exchanged for chlorine gas and the reaction was started. The chlorine gas flow rate was controlled to be 0.13 mol/hr. The water content of the L-type zeolite having the crystal size of at most 100 nm that had been prepared in Example 13 and calcinated at 400°C for 1 hour was 0.1% by weight.

After 8 hours, the orthoxylene conversion was 43%, and the production ratio of 4-chloro-orthoxylene/3-chloro-orthoxylene was 1.8.

(Comparative Example 5)

A 100-ml four-neck flask was equipped with a reflux condenser, a thermometer, a chlorine inlet tube, and a sampling duct. This was so designed that waste chlorine gas could be exhausted from the top of the reflux condenser and trapped in an aqueous sodium hydroxide solution. Heating it was effected in an oil bath.

53 g of orthoxylene (Sigma Aldrich Japan's first class grade chemical) that had been previously dried with Molecular Sieve 4A to have a water content of 20 ppm, and 2.5 g of a K-L-type zeolite (from Tosoh) that had been calcinated at 400°C for 1 hour and then cooled in a desiccator were put into the flask, and heated at 100°C with nitrogen gas being introduced thereinto through the chlorine gas inlet duct. After this was heated at 100°C, the nitrogen gas was exchanged for chlorine gas and the reaction was started. The chlorine gas flow rate was controlled to be 0.13 mol/hr. The water content of the K-L-type zeolite that had been calcinated at 400°C for 1 hour was 0.1% by weight.

After 8 hours, the orthoxylene conversion was 40%, and the production ratio of 4-chloro-orthoxylene/3-chloro-orthoxylene was 1.6.

(Example 17) Chlorination with a catalyst of L-type zeolite having the crystal size of at most 100 nm:

A 100-ml four-neck flask was equipped with a reflux condenser, a thermometer, a chlorine inlet tube, and a sampling duct. This was so designed that waste chlorine gas could be exhausted from the top of the reflux condenser and trapped in an aqueous sodium hydroxide solution. Heating it was effected in an oil bath.

12.74 g of orthoxylene (Sigma Aldrich Japan' s first class grade chemical) that had been previously dried with Molecular Sieve 4A to have a water content of 20 ppm, 45 ml of 1,2-dichloroethane (Sigma Aldrich Japan's first class grade chemical), and 2.54 g of the L-type zeolite having the crystal size of at most 100 nm that had been prepared in Example 14 and calcinated at 400°C for 1 hour and then cooled in a desiccator were put into the flask, and heated at 80°C with nitrogen gas being introduced thereinto through the chlorine gas inlet tube. After this was heated at 80°C, the nitrogen gas was mixed with chlorine gas and the reaction was started. The gas blend ratio was nitrogen gas/chlorine gas = 4 by volume. The chlorine gas flow rate was controlled to be 0.082 mol/hr. The water content of the L-type zeolite having the crystal size of at most 100 nm that had been prepared in Example 14 and calcinated at 400°C for 1 hour was 0.1 % by weight.

After 1 hour, the orthoxylene conversion was 46 %, and the production ratio of 4-chloro-orthoxylene/3-chloro-orthoxylene was 10.3.

(Comparative Example 6)

A 100-ml four-neck flask was equipped with a reflux condenser, a thermometer, a chlorine inlet tube, and a sampling duct. This was so designed that waste chlorine gas could be exhausted from the top of the reflux condenser and trapped in an aqueous sodium hydroxide solution. Heating it was effected in an oil bath.

12.74 g of orthoxylene (Sigma Aldrich Japan's first class grade chemical) that had been previously dried with Molecular Sieve 4A to have a water content of 20 ppm, 45 ml of 1,2-dichloroethane (Sigma Aldrich Japan's first class grade chemical), and 2.54 g of a K-L-type zeolite (from Tosoh) that had been calcinated at 400°C for 1 hour and then cooled in a desiccator were put into the flask, and heated at 80°C with nitrogen gas being introduced thereinto through the chlorine gas inlet duct. After this was heated at 80°C, the nitrogen gas was mixed with chlorine gas and the reaction was started. The gas blend ratio was nitrogen gas/chlorine gas = 4 by volume. The chlorine gas flow rate was controlled to be 0.082 mol/hr. The water content of the K-L-type zeolite that had been calcinated at 400°C for 1 hour was 0.1% by weight.

After 1 hour, the orthoxylene conversion was 43 %, and the production ratio of 4-chloro-orthoxylene/3-chloro-orthoxylene was 9.6.

(Example 18) Chlorination with a catalyst of L-type zeolite having the crystal size of at most 100 nm:

A 100-ml four-neck flask was equipped with a reflux condenser, a thermometer, a chlorine inlet tube, and a sampling duct. This was so designed that waste chlorine gas could be exhausted from the top of the reflux condenser and trapped in an aqueous sodium hydroxide solution. Heating it was effected in an oil bath.

16.96 g of orthoxylene (Sigma Aldrich Japan's first class grade chemical) that had been previously dried with Molecular Sieve 4A to have a water content of 20 ppm, 60 ml of 1,2-dichloroethane (Sigma Aldrich Japan's first class grade chemical), and 0.17 g of the L-type zeolite having the crystal size of at most 100 nm that had been prepared in Example 14 and calcinated at 400°C for 1 hour and then cooled in a desiccator were put into the flask, and heated at 80°C with nitrogen gas being introduced thereinto through the chlorine gas inlet tube. After this was heated at 80°C, the nitrogen gas was mixed with chlorine gas and the reaction was started. The gas blend ratio was nitrogen gas/chlorine gas = 4 by volume. The chlorine gas flow rate was controlled to be 0.026 mol/hr. The water content of the L-type zeolite having the crystal size of at most 100 nm that had been prepared in Example 14 and calcinated at 400°C for 1 hour was 0.1 % by weight.

After 6 hours, the orthoxylene conversion was 97 %, and the production ratio of 4-chloro-orthoxylene/3-chloro-orthoxylene was 10.4.

(Example 19) Preparation of fluorine-containing L-type zeolite having the crystal size of at most 100 nm:

3.22 g of anhydrous potassium fluoride and 60 ml of distilled water were added to 10 g (absolute dry) the L-type zeolite having the crystal size of at most 100 nm that had been prepared in Example 14, and stirred for 10 minutes (room temperature) and then filtered, and the resulting cake was dried overnight at 120°C.

(Example 20) Chlorination with a catalyst of fluorine-containing L-type zeolite having the crystal size of at most 100 nm:

A 100-ml four-neck flask was equipped with a reflux condenser, a thermometer, a chlorine inlet tube, and a sampling duct. This was so designed that waste chlorine gas could be exhausted from the top of the reflux condenser and trapped in an aqueous sodium hydroxide solution. Heating it was effected in an oil bath.

16.96 g of orthoxylene (Sigma Aldrich Japan' s first class grade chemical) that had been previously dried with Molecular Sieve 4A to have a water content of 20 ppm, 60 ml of 1,2-dichloroethane (Sigma Aldrich Japan's first class grade chemical), and 0.17 g of the fluorine-containing L-type zeolite having the crystal size of at most 100 nm that had been prepared in Example 19 and calcinated at 400°C for 1 hour and then cooled in a desiccator were put into the flask, and heated at 80°C with nitrogen gas being introduced thereinto through the chlorine gas inlet duct. After this was heated at 80°C, the nitrogen gas was mixed with chlorine gas and the reaction was started. The gas blend ratio was nitrogen gas/chlorine gas = 4 by volume. The chlorine gas flow rate was controlled to be 0.026 mol/hr. The water content of the fluorine-containing L-type zeolite having the crystal size of at most 100 nm that had been prepared in Example 19 and calcinated at 400°C for 1 hour was 0.1% by weight.

After 6 hours, the orthoxylene conversion was 98%, and the production ratio of 4-chloro-orthoxylene/3-chloro-orthoxylene was 11.7. After 7.25 hours, the orthoxylene conversion was over 100 %, and the production ratio of 4-chloro-orthoxylene/3-chloro-orthoxylene was 20.9. At the point at which the conversion was over 100%, the 4-chloro-orthoxylene selectivity significantly increased.

(Example 21) Chlorination with a catalyst of fluorine-containing L-type zeolite having the crystal size of at most 100 nm:

A 100-ml four-neck flask was equipped with a reflux condenser, a thermometer, a chlorine inlet tube, and a sampling duct. This was so designed that waste chlorine gas could be exhausted from the top of the reflux condenser and trapped in an aqueous sodium hydroxide solution. Heating it was effected in an oil bath.

16.96 g of orthoxylene (Sigma Aldrich Japan's first class grade chemical) that had been previously dried with Molecular Sieve 4A to have a water content of 20 ppm, 60 ml of 1,2-dichloroethane (Sigma Aldrich Japan's first class grade chemical) , and 0.17 g of the fluorine-containing L-type zeolite having the crystal size of at most 100 nm that had been prepared in Example 19 and calcinated at 165°C for 12 hours and then cooled in a desiccator were put into the flask, and heated at 80°C with nitrogen gas being introduced thereinto through the chlorine gas inlet tube. After this was heated at 80°C, the nitrogen gas was mixed with chlorine gas and the reaction was started. The gas blend ratio was nitrogen gas/chlorine gas = 4 by volume. The chlorine gas flow rate was controlled to be 0.026 mol/hr. The water content of the fluorine-containing L-type zeolite having the crystal size of at most 100 nm that had been prepared in Example 19 and calcinated at 165°C for 12 hours was 1.3% by weight.

After 6 hours, the orthoxylene conversion was 97%, and the production ratio of 4-chloro-orthoxylene/3-chloro-orthoxylene was 10.7.

(Example 22) Chlorination with a catalyst of fluorine-containing L-type zeolite having the crystal size of at most 100 nm:

A 100-ml four-neck flask was equipped with a reflux condenser, a thermometer, a chlorine inlet tube, and a sampling duct. This was so designed that waste chlorine gas could be exhausted from the top of the reflux condenser

and trapped in an aqueous sodium hydroxide solution. Heating it was effected in an oil bath.

16.96 g of orthoxylene (Sigma Aldrich Japan's first class grade chemical) that had been previously dried with Molecular Sieve 4A to have a water content of 20 ppm, and 0.17 g of the fluorine-containing L-type zeolite having the crystal size of at most 100 nm that had been prepared in Example 19 and calcinated at 165°C for 12 hours and then cooled in a desiccator were put into the flask, and heated at 80°C with nitrogen gas being introduced thereinto through the chlorine gas inlet duct. Then, this was heated at 130°C for 12 hours to remove water from the zeolite. Next, this was cooled to 80°C, and 60 ml of 1,2-dichloroethane (Sigma Aldrich Japan's first class grade chemical) was added to it, and the nitrogen gas was mixed with chlorine gas and the reaction was started. The gas blend ratio was nitrogen gas/chlorine gas = 4 by volume. The chlorine gas flow rate was controlled to be 0.026 mol/hr. The water content of the fluorine-containing L-type zeolite having the crystal size of at most 100 nm that had been prepared in Example 19 and calcinated at 165°C for 12 hours was 1.3 % by weight. Since the zeolite was heated in xylene at 130°C, the water content thereof during reaction would be lower than 1. 3 % by weight since this was not exposed at all to air after its heating at 130°C.

After 6 hours, the orthoxylene conversion was 97%, and the production ratio of 4-chloro-orthoxylene/3-chloro-orthoxylene was 11.4.

(Example 23) Chlorination with a catalyst of fluorine-containing L-type zeolite having the crystal size of at most 100 nm:

The same process as in Example 20 was carried out, in which, however, the orthoxylene chemical was directly used as it was. The water content of the orthoxylene chemical was measured and was 138 ppm.

After 6 hours, the orthoxylene conversion was 96%, and the production ratio of 4-chloro-orthoxylene/3-chloro-orthoxylene was 10.9.

(Example 24) Chlorination with a catalyst of fluorine-containing L-type zeolite:

A 100-ml four-neck flask was equipped with a reflux condenser, a thermometer, a chlorine inlet tube, and a sampling duct. This was so designed that waste chlorine gas could be exhausted from the top of the reflux condenser and trapped in an aqueous sodium hydroxide solution. Heating it was effected in an oil bath.

11.06 g of toluene (Kanto Chemical's special grade chemical), 45 ml of 1,2-dichloroethane (Sigma Aldrich Japan's first class grade chemical), and 2.54 g of the fluorine-containing K-L-type zeolite (from Tosoh) that had been prepared in Example 2 and calcinated at 400°C for 1 hour and then cooled in a desiccator were put into the flask, and heated at 40°C with nitrogen gas being introduced thereinto through the chlorine gas inlet tube. After this was heated at 40°C, the nitrogen gas was mixed with chlorine gas and the reaction was started. The gas blend ratio was nitrogen gas/chlorine gas = 4 by volume. The chlorine gas flow rate was controlled to be 0.06 mol/hr.

After 2 hours, the toluene conversion was 78 %, and the production ratio of para-chlorotoluene/(para-chlorotoluene + meta-chlorotoluene + ortho-chlorotoluene) was 10.9.

(Comparative Example 7)

A 100-ml four-neck flask was equipped with a reflux condenser, a thermometer, a chlorine inlet tube, and a sampling duct. This was so designed that waste chlorine gas could be exhausted from the top of the reflux condenser and trapped in an aqueous sodium hydroxide solution. Heating it was effected in an oil bath.

11.06 g of toluene (Kanto Chemical's special grade chemical), 45 ml of 1,2-dichloroethane (Sigma Aldrich Japan's first class grade chemical), and 2.54 g of a K-L-type zeolite (from Tosoh) that had been calcinated at 400°C for 1 hour and then cooled in a desiccator were put into the flask, and heated at 40°C with nitrogen gas being introduced thereinto through the chlorine gas inlet tube. After this was heated at 40°C, the nitrogen gas was mixed with chlorine gas and the reaction was started. The gas blend ratio was nitrogen gas/chlorine gas = 4 by volume. The chlorine gas flow rate was controlled to be 0.06 mol/hr.

After 2 hours, the toluene conversion was 76%, and the production ratio of para-chlorotoluene/(para-chlorotoluene + meta-chlorotoluene + ortho-chlorotoluene) was 9.8.

(Example 25) Chlorination with a catalyst of fluorine-containing L-type zeolite:

A 100-ml four-neck flask was equipped with a reflux condenser, a thermometer, a chlorine inlet tube, and a sampling duct. This was so designed that waste chlorine gas could be exhausted from the top of the reflux condenser and trapped in an aqueous sodium hydroxide solution. Heating it was effected in an oil bath.

11.06 g of toluene (Kanto Chemical's special grade chemical), 45 ml of 1,2-dichloroethane (Sigma Aldrich Japan's first class grade chemical), and 2.54 g of the fluorine-containing K-L-type zeolite (from Tosoh) that had been

prepared in Example 2 and calcinated at 400°C for 1 hour and then cooled in a desiccator were put into the flask, and heated at 80°C with nitrogen gas being introduced thereinto through the chlorine gas inlet duct. After this was heated at 80°C, the nitrogen gas was mixed with chlorine gas and the reaction was started. The gas blend ratio was nitrogen gas/chlorine gas = 4 by volume. The chlorine gas flow rate was controlled to be 0.06 mol/hr.

After 2 hours, the toluene conversion was 66 %, and the production ratio of para-chlorotoluene/(para-chlorotoluene + meta-chlorotoluene + ortho-chlorotoluene) was 8.5. After 3.5 hours, the toluene conversion was over 100 %, and the production ratio of para-chlorotoluene/(para-chlorotoluene + meta-chlorotoluene + ortho-chlorotoluene) was 17.3

(Comparative Example 8)

A 100-ml four-neck flask was equipped with a reflux condenser, a thermometer, a chlorine inlet tube, and a sampling duct. This was so designed that waste chlorine gas could be exhausted from the top of the reflux condenser and trapped in an aqueous sodium hydroxide solution. Heating it was effected in an oil bath.

11.06 g of toluene (Kanto Chemical's special grade chemical), 45 ml of 1, 2-dichloroethane (Sigma Aldrich Japan's first class grade chemical), and 2.54 g of a K-L-type zeolite (from Tosoh) that had been calcinated at 400°C for 1 hour and then cooled in a desiccator were put into the flask, and heated at 80°C with nitrogen gas being introduced thereinto through the chlorine gas inlet tube. After this was heated at 80°C, the nitrogen gas was mixed with chlorine gas and the reaction was started. The gas blend ratio was nitrogen gas/chlorine gas = 4 by volume. The chlorine gas flow rate was controlled to be 0.06 mol/hr.

After 2 hours, the toluene conversion was 68 %, and the production ratio of para-chlorotoluene/(para-chlorotoluene + meta-chlorotoluene + ortho-chlorotoluene) was 7.6.

(Example 26) Chlorination with a catalyst of fluorine-containing L-type zeolite:

A 100-ml four-neck flask was equipped with a reflux condenser, a thermometer, a chlorine inlet tube, and a sampling duct. This was so designed that waste chlorine gas could be exhausted from the top of the reflux condenser and trapped in an aqueous sodium hydroxide solution. Heating it was effected in an oil bath.

14.42 g of cumene (Kanto Chemical's special grade chemical), 45 ml of 1,2-dichloroethane (Sigma Aldrich Japan's first class grade chemical), and 2.54 g of the fluorine-containing K-L-type zeolite (from Tosoh) that had been prepared in Example 2 and calcinated at 400°C for 1 hour and then cooled in a desiccator were put into the flask, and heated at 40°C with nitrogen gas being introduced thereinto through the chlorine gas inlet duct. After this was heated at 40°C, the nitrogen gas was mixed with chlorine gas and the reaction was started. The gas blend ratio was nitrogen gas/chlorine gas = 4 by volume. The chlorine gas flow rate was controlled to be 0.06 mol/hr.

After 2 hours, the cumene conversion was 76 %, and the production ratio of para-chlorocumene/(para-chlorocumene + meta-chlorocumene + ortho-chlorocumene) was 51.6. After 3 hours, the cumene conversion was over 100%, and the production ratio of para-chlorocumene/(para-chlorocumene + meta-chlorocumene + ortho-chlorocumene) was 106.8.

(Comparative Example 9)

A 100-ml four-neck flask was equipped with a reflux condenser, a thermometer, a chlorine inlet tube, and a sampling duct. This was so designed that waste chlorine gas could be exhausted from the top of the reflux condenser and trapped in an aqueous sodium hydroxide solution. Heating it was effected in an oil bath.

14.42 g of cumene (Kanto Chemical's special grade chemical), 45 ml of 1,2-dichloroethane (Sigma Aldrich Japan's first class grade chemical), and 2.54 g of a K-L-type zeolite (from Tosoh) that had been calcinated at 400°C for 1 hour and then cooled in a desiccator were put into the flask, and heated at 40°C with nitrogen gas being introduced thereinto through the chlorine gas inlet tube. After this was heated at 40°C, the nitrogen gas was mixed with chlorine gas and the reaction was started. The gas blend ratio was nitrogen gas/chlorine gas = 4 by volume. The chlorine gas flow rate was controlled to be 0.06 mol/hr.

After 2 hours, the cumene conversion was 75%, and the production ratio of para-chlorocumene/(para-chlorocumene + meta-chlorocumene + ortho-chlorocumene) was 25.4.

(Example 27) Chlorination with a catalyst of fluorine-containing L-type zeolite:

A 100-ml four-neck flask was equipped with a reflux condenser, a thermometer, a chlorine inlet tube, and a sampling duct. This was so designed that waste chlorine gas could be exhausted from the top of the reflux condenser and trapped in an aqueous sodium hydroxide solution. Heating it was effected in an oil bath.

14.42 g of cumene (Kanto Chemical's special grade chemical), 45 ml of 1,2-dichloroethane (Sigma Aldrich Japan's first class grade chemical), and 2.54 g of the fluorine-containing K-L-type zeolite (from Tosoh) that had been prepared in Example 2 and calcinated at 400°C for 1 hour and then cooled in a desiccator were put into the flask, and heated at 80°C with nitrogen gas being introduced thereinto through the chlorine gas inlet duct. After this was heated at 80°C, the nitrogen gas was mixed with chlorine gas and the reaction was started. The gas blend ratio was nitrogen gas/chlorine gas = 4 by volume. The chlorine gas flow rate was controlled to be 0.06 mol/hr.

After 2 hours, the cumene conversion was 76%, and the production ratio of para-chlorocumene/(para-chlorocumene + meta-chlorocumene + ortho-chlorocumene) was 34.7.

(Comparative Example 10)

A 100-ml four-neck flask was equipped with a reflux condenser, a thermometer, a chlorine inlet tube, and a sampling duct. This was so designed that waste chlorine gas could be exhausted from the top of the reflux condenser and trapped in an aqueous sodium hydroxide solution. Heating it was effected in an oil bath.

14.42 g of cumene (Kanto Chemical's special grade chemical), 45 ml of 1,2-dichloroethane (Sigma Aldrich Japan's first class grade chemical), and 2.54 g of a K-L-type zeolite (from Tosoh) that had been calcinated at 400°C for 1 hour and then cooled in a desiccator were put into the flask, and heated at 80°C with nitrogen gas being introduced thereinto through the chlorine gas inlet tube. After this was heated at 80°C, the nitrogen gas was mixed with chlorine gas and the reaction was started. The gas blend ratio was nitrogen gas/chlorine gas = 4 by volume. The chlorine gas flow rate was controlled to be 0.06 mol/hr.

After 2 hours, the cumene conversion was 75%, and the production ratio of para-chlorocumene/(para-chlorocumene + meta-chlorocumene + ortho-chlorocumene) was 17.1.

(Example 28) Chlorination with a catalyst of fluorine-containing L-type zeolite:

A 100-ml four-neck flask was equipped with a reflux condenser, a thermometer, a chlorine inlet tube, and a sampling duct. This was so designed that waste chlorine gas could be exhausted from the top of the reflux condenser and trapped in an aqueous sodium hydroxide solution. Heating it was effected in an oil bath.

16.11 g of tertiary butylbenzene (Kanto Chemical's special grade chemical), 45 ml of 1,2-dichloroethane (Sigma Aldrich Japan's first class grade chemical), and 2.54 g of the fluorine-containing K-L-type zeolite (from Tosoh) that had been prepared in Example 2 and calcinated at 400°C for 1 hour and then cooled in a desiccator were put into the flask, and heated at 40°C with nitrogen gas being introduced thereinto through the chlorine gas inlet duct. After this was heated at 40°C, the nitrogen gas was mixed with chlorine gas and the reaction was started. The gas blend ratio was nitrogen gas/chlorine gas = 4 by volume. The chlorine gas flow rate was controlled to be 0.06 mol/hr.

After 2 hours, the tertiary butylbenzene conversion was 80 %, and the production ratio of para-chloro-tert-butylbenzene/(para-chloro-tert-butylbenzene + meta-chloro-tert-butylbenzene + ortho-chloro-tert-butylbenzene) was 201.

(Comparative Example 11)

A 100-ml four-neck flask was equipped with a reflux condenser, a thermometer, a chlorine inlet tube, and a sampling duct. This was so designed that waste chlorine gas could be exhausted from the top of the reflux condenser and trapped in an aqueous sodium hydroxide solution. Heating it was effected in an oil bath.

16.11 g of tertiary butylbenzene (Kanto Chemical's special grade chemical), 45 ml of 1,2-dichloroethane (Sigma Aldrich Japan's first class grade chemical), and 2.54 g of a K-L-type zeolite (from Tosoh) that had been calcinated at 400°C for 1 hour and then cooled in a desiccator were put into the flask, and heated at 40°C with nitrogen gas being introduced thereinto through the chlorine gas inlet tube. After this was heated at 40°C, the nitrogen gas was mixed with chlorine gas and the reaction was started. The gas blend ratio was nitrogen gas/chlorine gas = 4 by volume. The chlorine gas flow rate was controlled to be 0.06 mol/hr.

After 2 hours, the tertiary butylbenzene conversion was 78%, and the production ratio of para-chloro-tert-butylbenzene/(para-chloro-tert-butylbenzene + meta-chloro-tert-butylbenzene + ortho-chloro-tert-butylbenzene) was 99.

(Example 29) Chlorination with a catalyst of fluorine-containing L-type zeolite:

A 100-ml four-neck flask was equipped with a reflux condenser, a thermometer, a chlorine inlet tube, and a sampling duct. This was so designed that waste chlorine gas could be exhausted from the top of the reflux condenser and trapped in an aqueous sodium hydroxide solution. Heating it was effected in an oil bath.

16.11 g of tertiary butylbenzene (Kanto Chemical's special grade chemical), 45 ml of 1,2-dichloroethane (Sigma Aldrich Japan's first class grade chemical), and 2.54 g of the fluorine-containing K-L-type zeolite (from Tosoh) that had been prepared in Example 2 and calcinated at 400°C for 1 hour and then cooled in a desiccator were put into the flask, and heated at 80°C with nitrogen gas being introduced thereinto through the chlorine gas inlet duct. After this was heated at 80°C, the nitrogen gas was mixed with chlorine gas and the reaction was started. The gas blend ratio was nitrogen gas/chlorine gas = 4 by volume. The chlorine gas flow rate was controlled to be 0.06 mol/hr.

After 2 hours, the tertiary butylbenzene conversion was 71%, and the production ratio of para-chloro-tert-butylbenzene/(para-chloro-tert-butylbenzene + meta-chloro-tert-butylbenzene + ortho-chloro-tert-butylbenzene) was 112.

(Comparative Example 12)

A 100-ml four-neck flask was equipped with a reflux condenser, a thermometer, a chlorine inlet tube, and a sampling duct. This was so designed that waste chlorine gas could be exhausted from the top of the reflux condenser and trapped in an aqueous sodium hydroxide solution. Heating it was effected in an oil bath.

16.11 g of tertiary butylbenzene (Kanto Chemical's special grade chemical), 45 ml of 1,2-dichloroethane (Sigma Aldrich Japan's first class grade chemical), and 2.54 g of a K-L-type zeolite (from Tosoh) that had been calcinated at 400°C for 1 hour and then cooled in a desiccator were put into the flask, and heated at 80°C with nitrogen gas being introduced thereinto through the chlorine gas inlet tube. After this was heated at 80°C, the nitrogen gas was mixed with chlorine gas and the reaction was started. The gas blend ratio was nitrogen gas/chlorine gas = 4 by volume. The chlorine gas flow rate was controlled to be 0.06 mol/hr.

After 2 hours, the tertiary butylbenzene conversion was 72 %, and the production ratio of para-chloro-tert-butylbenzene/(para-chloro-tert-butylbenzene + meta-chloro-tert-butylbenzene + ortho-chloro-tert-butylbenzene) was 49.

(Example 30) Chlorination with a catalyst of fluorine-containing L-type zeolite:

A 100-ml four-neck flask was equipped with a reflux condenser, a thermometer, a chlorine inlet tube, and a sampling duct. This was so designed that waste chlorine gas could be exhausted from the top of the reflux condenser and trapped in an aqueous sodium hydroxide solution. Heating it was effected in an oil bath.

13.51 g of chlorobenzene (Kanto Chemical's special grade chemical), 45 ml of 1,2-dichloroethane (Sigma Aldrich Japan's first class grade chemical), and 2.54 g of the fluorine-containing K-L-type zeolite (from Tosoh) that had been prepared in Example 2 and calcinated at 400°C for 1 hour and then cooled in a desiccator were put into the flask, and heated at 40°C with nitrogen gas being introduced thereinto through the chlorine gas inlet duct. After this was heated at 40°C, the nitrogen gas was mixed with chlorine gas and the reaction was started. The gas blend ratio was nitrogen gas/chlorine gas = 4 by volume. The chlorine gas flow rate was controlled to be 0.06 mol/hr.

After 2 hours, the chlorobenzene conversion was 62%, and the production ratio of para-dichlorobenzene/(para-dichlorobenzene + meta-dichlorobenzene + ortho-dichlorobenzene) was 65.7.

(Comparative Example 13)

A 100-ml four-neck flask was equipped with a reflux condenser, a thermometer, a chlorine inlet tube, and a sampling duct. This was so designed that waste chlorine gas could be exhausted from the top of the reflux condenser and trapped in an aqueous sodium hydroxide solution. Heating it was effected in an oil bath.

13.51 g of chlorobenzene (Kanto Chemical's special grade chemical), 45 ml of 1,2-dichloroethane (Sigma Aldrich Japan's first class grade chemical), and 2.54 g of a K-L-type zeolite (from Tosoh) that had been calcinated at 400°C for 1 hour and then cooled in a desiccator were put into the flask, and heated at 40°C with nitrogen gas being introduced thereinto through the chlorine gas inlet tube. After this was heated at 40°C, the nitrogen gas was mixed with chlorine gas and the reaction was started. The gas blend ratio was nitrogen gas/chlorine gas = 4 by volume. The chlorine gas flow rate was controlled to be 0.06 mol/hr.

After 2 hours, the chlorobenzene conversion was 60%, and the production ratio of para-dichlorobenzene/(para-dichlorobenzene + meta-dichlorobenzene + ortho-dichlorobenzene) was 20.5.

(Example 31) Chlorination with a catalyst of fluorine-containing L-type zeolite:

A 100-ml four-neck flask was equipped with a reflux condenser, a thermometer, a chlorine inlet tube, and a sampling duct. This was so designed that waste chlorine gas could be exhausted from the top of the reflux condenser and trapped in an aqueous sodium hydroxide solution. Heating it was effected in an oil bath.

13.51 g of chlorobenzene (Kanto Chemical' s special grade chemical), 45 ml of 1,2-dichloroethane (Sigma Aldrich Japan's first class grade chemical), and 2.54 g of the fluorine-containing K-L-type zeolite (from Tosoh) that had been prepared in Example 2 and calcinated at 400°C for 1 hour and then cooled in a desiccator were put into the flask, and heated at 80°C with nitrogen gas being introduced thereinto through the chlorine gas inlet duct. After this was heated at 80°C, the nitrogen gas was mixed with chlorine gas and the reaction was started. The gas blend ratio was nitrogen gas/chlorine gas = 4 by volume. The chlorine gas flow rate was controlled to be 0.06 mol/hr.
After 2 hours, the chlorobenzene conversion was 52%, and the production ratio of para-dichlorobenzene/ (para-dichlorobenzene + meta-dichlorobenzene + ortho-dichlorobenzene) was 44.5.

(Comparative Example 14)

A 100-ml four-neck flask was equipped with a reflux condenser, a thermometer, a chlorine inlet tube, and a sampling duct. This was so designed that waste chlorine gas could be exhausted from the top of the reflux condenser and trapped in an aqueous sodium hydroxide solution. Heating it was effected in an oil bath.
13.51 g of chlorobenzene (Kanto Chemical's special grade chemical), 45 ml of 1,2-dichloroethane (Sigma Aldrich Japan's first class grade chemical), and 2.54 g of a K-L-type zeolite (from Tosoh) that had been calcinated at 400°C for 1 hour and then cooled in a desiccator were put into the flask, and heated at 80°C with nitrogen gas being introduced thereinto through the chlorine gas inlet tube. After this was heated at 80°C, the nitrogen gas was mixed with chlorine gas and the reaction was started. The gas blend ratio was nitrogen gas/chlorine gas = 4 by volume. The chlorine gas flow rate was controlled to be 0.06 mol/hr.
After 2 hours, the chlorobenzene conversion was 55%, and the production ratio of para-dichlorobenzene/ (para-dichlorobenzene + meta-dichlorobenzene + ortho-dichlorobenzene) was 10.5.

(Example 32) Chlorination with a catalyst of fluorine-containing L-type zeolite:

A 100-ml four-neck flask was equipped with a reflux condenser, a thermometer, a chlorine inlet tube, and a sampling duct. This was so designed that waste chlorine gas could be exhausted from the top of the reflux condenser and trapped in an aqueous sodium hydroxide solution. Heating it was effected in an oil bath.
15.19 g of ortho-chlorotoluene (Kanto Chemical's special grade chemical), 45 ml of 1,2-dichloroethane (Sigma Aldrich Japan's first class grade chemical), and 2.54 g of the fluorine-containing K-L-type zeolite (from Tosoh) that had been prepared in Example 2 and calcinated at 400°C for 1 hour and then cooled in a desiccator were put into the flask, and heated at 80°C with nitrogen gas being introduced thereinto through the chlorine gas inlet duct. After this was heated at 80°C, the nitrogen gas was mixed with chlorine gas and the reaction was started. The gas blend ratio was nitrogen gas/chlorine gas = 4 by volume. The chlorine gas flow rate was controlled to be 0.06 mol/hr.
After 2 hours, the ortho-chlorotoluene conversion was 69%, and the production ratio of 2,5-dichlorotoluene to dichlorotoluene isomers was 2.5.

(Comparative Example 15)

A 100-ml four-neck flask was equipped with a reflux condenser, a thermometer, a chlorine inlet tube, and a sampling duct. This was so designed that waste chlorine gas could be exhausted from the top of the reflux condenser and trapped in an aqueous sodium hydroxide solution. Heating it was effected in an oil bath.
15.19 g of ortho-chlorotoluene (Kanto Chemical's special grade chemical), 45 ml of 1,2-dichloroethane (Sigma Aldrich Japan's first class grade chemical), and 2.54 g of a K-L-type zeolite (from Tosoh) that had been calcinated at 400°C for 1 hour and then cooled in a desiccator were put into the flask, and heated at 80°C with nitrogen gas being introduced thereinto through the chlorine gas inlet tube. After this was heated at 80°C, the nitrogen gas was mixed with chlorine gas and the reaction was started. The gas blend ratio was nitrogen gas/chlorine gas = 4 by volume. The chlorine gas flow rate was controlled to be 0.06 mol/hr.
After 2 hours, the ortho-chlorotoluene conversion was 65 %, and the production ratio of 2,5-dichlorotoluene to dichlorotoluene isomers was 1.9.

(Example 33) Chlorination with a catalyst of fluorine-containing L-type zeolite:

A 100-ml four-neck flask was equipped with a reflux condenser, a thermometer, a chlorine inlet tube, and a sampling duct. This was so designed that waste chlorine gas could be exhausted from the top of the reflux condenser and trapped in an aqueous sodium hydroxide solution. Heating it was effected in an oil bath.
15.19 g of meta-chlorotoluene (Kanto Chemical's special grade chemical), 45 ml of 1,2-dichloroethane (Sigma Aldrich Japan's first class grade chemical), and 2.54 g of the fluorine-containing K-L-type zeolite (from Tosoh) that had

been prepared in Example 2 and calcinated at 400°C for 1 hour and then cooled in a desiccator were put into the flask, and heated at 80°C with nitrogen gas being introduced thereinto through the chlorine gas inlet duct. After this was heated at 80°C, the nitrogen gas was mixed with chlorine gas and the reaction was started. The gas blend ratio was nitrogen gas/chlorine gas = 4 by volume. The chlorine gas flow rate was controlled to be 0.06 mol/hr.

After 2 hours, the meta-chlorotoluene conversion was 69%, and the production ratio of 2,5-dichlorotoluene to dichlorotoluene isomers was 3.1.

(Comparative Example 16)

A 100-ml four-neck flask was equipped with a reflux condenser, a thermometer, a chlorine inlet tube, and a sampling duct. This was so designed that waste chlorine gas could be exhausted from the top of the reflux condenser and trapped in an aqueous sodium hydroxide solution. Heating it was effected in an oil bath.

15.19 g of meta-chlorotoluene (Kanto Chemical's special grade chemical), 45 ml of 1,2-dichloroethane (Sigma Aldrich Japan's first class grade chemical), and 2.54 g of a K-L-type zeolite (from Tosoh) that had been calcinated at 400°C for 1 hour and then cooled in a desiccator were put into the flask, and heated at 80°C with nitrogen gas being introduced thereinto through the chlorine gas inlet tube. After this was heated at 80°C, the nitrogen gas was mixed with chlorine gas and the reaction was started. The gas blend ratio was nitrogen gas/chlorine gas = 4 by volume. The chlorine gas flow rate was controlled to be 0.06 mol/hr.

After 2 hours, the meta-chlorotoluene conversion was 61%, and the production ratio of 2,5-dichlorotoluene to dichlorotoluene isomers was 2.4.

(Example 34) Chlorination with a catalyst of fluorine-containing L-type zeolite:

A 100-ml four-neck flask was equipped with a reflux condenser, a thermometer, a chlorine inlet tube, and a sampling duct. This was so designed that waste chlorine gas could be exhausted from the top of the reflux condenser and trapped in an aqueous sodium hydroxide solution. Heating it was effected in an oil bath.

15.19 g of para-chlorotoluene (Kanto Chemical's special grade chemical), 45 ml of 1,2-dichloroethane (Sigma Aldrich Japan's first class grade chemical), and 2.54 g of the fluorine-containing K-L-type zeolite (from Tosoh) that had been prepared in Example 2 and calcinated at 400°C for 1 hour and then cooled in a desiccator were put into the flask, and heated at 80°C with nitrogen gas being introduced thereinto through the chlorine gas inlet duct. After this was heated at 80°C, the nitrogen gas was mixed with chlorine gas and the reaction was started. The gas blend ratio was nitrogen gas/chlorine gas = 4 by volume. The chlorine gas flow rate was controlled to be 0.06 mol/hr.

After 2 hours, the para-chlorotoluene conversion was 24%, and the production ratio of 2,4-dichlorotoluene to dichlorotoluene isomers was 2.6

(Comparative Example 17)

A 100-ml four-neck flask was equipped with a reflux condenser, a thermometer, a chlorine inlet tube, and a sampling duct. This was so designed that waste chlorine gas could be exhausted from the top of the reflux condenser and trapped in an aqueous sodium hydroxide solution. Heating it was effected in an oil bath.

15.19 g of para-chlorotoluene (Kanto Chemical's special grade chemical), 45 ml of 1,2-dichloroethane (Sigma Aldrich Japan's first class grade chemical), and 2.54 g of a K-L-type zeolite (from Tosoh) that had been calcinated at 400°C for 1 hour and then cooled in a desiccator were put into the flask, and heated at 80°C with nitrogen gas being introduced thereinto through the chlorine gas inlet tube. After this was heated at 80°C, the nitrogen gas was mixed with chlorine gas and the reaction was started. The gas blend ratio was nitrogen gas/chlorine gas = 4 by volume. The chlorine gas flow rate was controlled to be 0.06 mol/hr.

After 2 hours, the para-chlorotoluene conversion was 20%, and the production ratio of 2,4-dichlorotoluene to dichlorotoluene isomers was 1.8.

(Example 35) Chlorination with a catalyst of fluorine-containing L-type zeolite:

A 100-ml four-neck flask was equipped with a reflux condenser, a thermometer, a chlorine inlet tube, and a sampling duct. This was so designed that waste chlorine gas could be exhausted from the top of the reflux condenser and trapped in an aqueous sodium hydroxide solution. Heating it was effected in an oil bath.

17.64 g of meta-dichlorobenzene (Tokyo Chemical Industry's special grade chemical), 45 ml of 1,2-dichloroethane (Sigma Aldrich Japan's first class grade chemical), and 2.54 g of the fluorine-containing K-L-type zeolite (from Tosoh) that had been prepared in Example 2 and calcinated at 400°C for 1 hour and then cooled in a desiccator were put into the flask, and heated at 80°C with nitrogen gas being introduced thereinto through the chlorine gas inlet duct.

After this was heated at 80°C, the nitrogen gas was mixed with chlorine gas and the reaction was started. The gas blend ratio was nitrogen gas/chlorine gas = 4 by volume. The chlorine gas flow rate was controlled to be 0.06 mol/hr.

After 4 hours, the meta-dichlorobenzene conversion was 6%, and the production ratio of 1,2,4-trichlorobenzene to trichlorobenzene isomers was 54.

(Comparative Example 18)

A 100-ml four-neck flask was equipped with a reflux condenser, a thermometer, a chlorine inlet tube, and a sampling duct. This was so designed that waste chlorine gas could be exhausted from the top of the reflux condenser and trapped in an aqueous sodium hydroxide solution. Heating it was effected in an oil bath.

17.64 g of meta-dichlorobenzene (Tokyo Chemical Industry' s special grade chemical), 45 ml of 1, 2-dichloroethane (Sigma Aldrich Japan's first class grade chemical), and 2.54 g of a K-L-type zeolite (from Tosoh) that had been calcinated at 400°C for 1 hour and then cooled in a desiccator were put into the flask, and heated at 80°C with nitrogen gas being introduced thereinto through the chlorine gas inlet tube. After this was heated at 80°C, the nitrogen gas was mixed with chlorine gas and the reaction was started. The gas blend ratio was nitrogen gas /chlorine gas = 4 by volume. The chlorine gas flow rate was controlled to be 0.06 mol/hr.

After 4 hours, the meta-dichlorobenzene conversion was 5 %, and the production ratio of 1,2,4-trichlorobenzene to trichlorobenzene isomers was 19.5.

(Example 36) Chlorination with a catalyst of fluorine-containing L-type zeolite:

A 100-ml four-neck flask was equipped with a reflux condenser, a thermometer, a chlorine inlet tube, and a sampling duct. This was so designed that waste chlorine gas could be exhausted from the top of the reflux condenser and trapped in an aqueous sodium hydroxide solution. Heating it was effected in an oil bath.

52.92 g of meta-dichlorobenzene (Tokyo Chemical Industry's special grade chemical), and 2.54 g of the fluorine-containing K-L-type zeolite (from Tosoh) that had been prepared in Example 2 and calcinated at 400°C for 1 hour and then cooled in a desiccator were put into the flask, and heated at 120°C with nitrogen gas being introduced thereinto through the chlorine gas inlet duct. After this was heated at 120°C, the nitrogen gas was mixed with chlorine gas and the reaction was started. The gas blend ratio was nitrogen gas/chlorine gas = 4 by volume. The chlorine gas flow rate was controlled to be 0.06 mol/hr.

After 4 hours, the meta-dichlorobenzene conversion was 54%, and the production ratio of 1,2,4-trichlorobenzene to trichlorobenzene isomers was 19.8.

(Comparative Example 19)

A 100-ml four-neck flask was equipped with a reflux condenser, a thermometer, a chlorine inlet tube, and a sampling duct. This was so designed that waste chlorine gas could be exhausted from the top of the reflux condenser and trapped in an aqueous sodium hydroxide solution. Heating it was effected in an oil bath.

52.92 g of meta-dichlorobenzene (Tokyo Chemical Industry's special grade chemical), and 2.54 g of a K-L-type zeolite (from Tosoh) that had been calcinated at 400°C for 1 hour and then cooled in a desiccator were put into the flask, and heated at 120°C with nitrogen gas being introduced thereinto through the chlorine gas inlet tube. After this was heated at 120°C, the nitrogen gas was mixed with chlorine gas and the reaction was started. The gas blend ratio was nitrogen gas/chlorine gas = 4 by volume. The chlorine gas flow rate was controlled to be 0.06 mol/hr.

After 4 hours, the meta-dichlorobenzene conversion was 50%, and the production ratio of 1,2,4-trichlorobenzene to trichlorobenzene isomers was 11.1.

(Reference Example 1) Preparation of mixture of chloroalkylbenzene derivative with an impurity of α-chloroalkylbenzene (benzyl chloride) derivative:

A 200-ml four-neck flask was equipped with a reflux condenser, a thermometer, a chlorine inlet tube, and a sampling duct. This was so designed that waste chlorine gas (hydrochloric acid gas) could be exhausted from the top of the reflux condenser and trapped in an aqueous sodium hydroxide solution. Heating it was effected in an oil bath.

17.1 g of orthoxylene orthoxylene (Sigma Aldrich Japan's first class grade chemical) that had been previously dried with Molecular Sieve 4A to have a water content of 20 ppm, 74.8 g of 1, 2-dichloroethane (Sigma Aldrich Japan's first class grade chemical), and 3.43 g of the fluorine-containing K-L-type zeolite (from Tosoh) that had been prepared in Example 1 and calcinated at 400°C for 1 hour and then cooled in a desiccator were put into the flask, and heated at 80°C with nitrogen gas being introduced thereinto through the chlorine gas inlet tube. After this was heated at 80°C, the nitrogen gas was exchanged for chlorine gas and the reaction was started. The chlorine gas flow rate was controlled

to be about 0.03 mol/hr.

After 3 hours and 40 minutes, the chlorine gas introduction was stopped, the flask was bubbled with nitrogen for 1 hour, then the reaction solution was cooled to room temperature, and the catalyst was taken out through filtration. The reaction solution (filtrate) was concentrated, and 31 g of a crude product having a composition ratio shown in Table 1 and Table 2 was obtained.

(Reference Example 2) Preparation of mixture of chloroalkylbenzene derivative with an impurity of α-chloroalkylbenzene (benzyl chloride) derivative:

A 100-ml four-neck flask was equipped with a reflux condenser, a thermometer, a chlorine inlet tube, and a sampling duct. This was so designed that waste chlorine gas could be exhausted from the top of the reflux condenser and trapped in an aqueous sodium hydroxide solution. Heating it was effected in an oil bath.

12.7 g of orthoxylene (Sigma Aldrich Japan's first class grade chemical) that had been previously dried with Molecular Sieve 4A to have a water content of 20 ppm, 30 ml of 1,2-dichloroethane (Sigma Aldrich Japan's first class grade chemical), 0.56 g of 1,4-dioxane (Katayama Chemical Industry's special grade chemical), and 1.69 g of a K-L-type zeolite (from Tosoh) that had been calcinated at 400°C for 1 hour and then cooled in a desiccator were put into the flask, and heated at 80°C with nitrogen gas being introduced thereinto through the chlorine gas inlet tube. After this was heated at 80°C, the nitrogen gas was exchanged for chlorine gas and the reaction was started. The chlorine gas flow rate was controlled to be about 0.04 mol/hr.

After 6 hours and 45 minutes , the chlorine gas introduction was stopped, the flask was bubbled with nitrogen for 1 hour, then the reaction solution was cooled to room temperature, and the catalyst was taken out through filtration. The reaction solution (filtrate) was concentrated, and 23 g of a product having a composition ratio shown in Table 1 and Table 2 was obtained.

(Reference Example 3) Preparation of mixture of chloroalkylbenzene derivative with an impurity of α-chloroalkylbenzene (benzyl chloride) derivative:

A 1000-ml four-neck flask was equipped with a reflux condenser, a thermometer, a chlorine inlet tube, and a sampling duct. This was so designed that waste chlorine gas could be exhausted from the top of the reflux condenser and trapped in an aqueous sodium hydroxide solution. Heating it was effected in an oil bath.

89.18 g of orthoxylene (Sigma Aldrich Japan' s first class grade chemical) that had been previously dried with Molecular Sieve 4A to have a water content of 20 ppm, 315 ml of 1,2-dichloroethane (Sigma Aldrich Japan's first class grade chemical), and 17 . 8 g of the fluorine-containing K-L-type zeolite (from Tosoh) that had been prepared in Example 1 and calcinated at 400°C for 1 hour and then cooled in a desiccator were put into the flask, and heated at 80°C with nitrogen gas being introduced thereinto through the chlorine gas inlet tube. After this was heated at 80°C, the nitrogen gas was exchanged for chlorine gas and the reaction was started. The chlorine gas flow rate was controlled to be about 0.82 mol/hr.

After 1 hour and 45 minutes, the chlorine gas introduction was stopped, the flask was bubbled with nitrogen for 1 hour, then the reaction solution was cooled to room temperature, and the catalyst was taken out through filtration. The reaction solution (filtrate) was concentrated, and 144 g of a product having a composition ratio shown in Table 1 and Table 2 was obtained.

(Comparative Example 20) Decomposition of α-chloroalkylbenzene derivative with Zn:

25 ml of ethanol (Nacalai Tesuque's first class grade chemical) was added to 13.5 g of the crude product obtained in Reference Example 2, and then 25 ml of concentrated hydrochloric acid (Kanto Chemical' s first class grade chemical) was gradually introduced into it. While the reaction solution was vigorously stirred, 6.17 g of activated zinc dust (Kanto Chemical's special grade chemical) was gradually added to it. After 4 hours, the reaction solution was analyzed, and the reaction was not completed. Accordingly, 25 ml of concentrated hydrochloric acid and 3.3 g of zinc dust were further added to the system, and the reaction completion took further 1.5 hours. 30 ml of chloroform (Kanto Chemical's first class grade chemical) was added to the reaction solution, and zinc dust was filtered off and subjected to liquid-liquid separation. The organic layer was washed with aqueous sodium bicarbonate solution and water, and then dried with anhydrous sodium sulfate (Sigma Aldrich Japan's first class grade chemical) for overnight. Sodium sulfate was removed through filtration, and the filtrate was concentrated to obtain 13 g of a product having a composition ratio shown in Table 2.

Tables 1 and 2 confirm that α-chloro-orthoxylene was reductively decomposed into orthoxylene.

(Example 37) Decomposition of α-chloroalkylbenzene derivative with a very small quantity of Fe:

7.3 mg of Fe (Katayama Chemical's special grade chemical) was added to 2.07 g of the crude product obtained in Reference Example 1 and stirred at 160°C for 90 minutes, which resulted in completion of selective decomposition of the compound. From Tables 1 and 2, it is understood that, as compared with the conventional method (Comparative Example 20), the amount of the metal to be used in this method could be much reduced and the selective decomposition could be attained in a more simplified manner for a shorter period of time.

(Example 38) Decomposition of α-chloroalkylbenzene derivative with a very small quantity of $FeCl_3$:

1. 5 mg of anhydrous $FeCl_3$ (Kishida Chemical's first class grade chemical) was added to 1.02 g of the crude product obtained in Reference Example 1 and stirred at 160°C for 10 minutes, which resulted in completion of selective decomposition of the compound. From Tables 1 and 2, it is understood that, as compared with Example 37, the amount of the metal to be used in this method could be reduced to at most 1/2, and the selective decomposition could be attained for a shorter period of time.

(Example 39) Decomposition of α-chloroalkylbenzene derivative with a very small quantity of $FeCl_3$:

2.0 mg of anhydrous $FeCl_3$ (Kishida Chemical's first class grade chemical) was added to 2.00 g of the crude product obtained in Reference Example 1 and stirred at 100°C for 10 minutes, which resulted in completion of selective decomposition of the compound. From Tables 1 and 2, it is understood that, as compared with Example 38, the amount of the metal to be used in this method could be reduced more, and the selective decomposition could be attained at a lower temperature.

(Example 40) Decomposition of α-chloroalkylbenzene derivative with a very small quantity of $FeCl_3$:

7. 8 mg of anhydrous $FeCl_3$ (Kishida Chemical's first class grade chemical) was added to 12.2 g of the crude product obtained in Reference Example 1 and stirred at room temperature for 280 minutes, but selective decomposition of the compound was as yet incomplete. From Tables 1 and 2, it is understood that the reaction temperature is preferably higher than room temperature.

(Example 41) Decomposition of α-chloroalkylbenzene derivative with a very small quantity of $FeCl_3$:

7. 8 mg of anhydrous $FeCl_3$ (Kishida Chemical's first class grade chemical) was added to 12.2 g of the crude product obtained in Reference Example 1 and stirred at 100°C for 20 minutes, which resulted in completion of selective decomposition of the compound. From Tables 1 and 2, it is understood that, as compared with Example 39, the amount of the metal to be used in this method could be reduced more in completing the selective decomposition of the compound.

(Example 42) Decomposition of α-chloroalkylbenzene derivative with a very small quantity of $FeCl_3$:

The completely-reacted solution in Example 41 was further heated up to 160°C and stirred still under heat for 6 hours and 40 minutes, but remarkable decomposition of the chloroalkylbenzene derivative to be purified did not almost occur. Since the compound is stable to heat, it is understood from Tables 1 and 2 that, after the compound is decomposed, it may be purified through distillation.

(Example 43) Decomposition of α-chloroalkylbenzene derivative with a very small quantity of $AlCl_3$:

2.5 mg of anhydrous $AlCl_3$ (Katayama Chemical's special grade chemical) was added to 2.03 g of the crude product obtained in Reference Example 1 and stirred at room temperature for 120 minutes, but the selective decomposition of the compound was still insufficient in some degree. Table 1 and Table 2 show the composition of the reaction mixture.

(Comparative Example 21) 15-plates distillation after (base) treatment before reaction and distillation:

The crude product obtained in Reference Example 3 was washed with an aqueous solution of saturated sodium carbonate (Kanto Chemical's first class grade chemical), and subjected to liquid-liquid separation. This was dried with anhydrous sodium sulfate (Nacalai Tesuque's special grade chemical) added to it, then filtered, and distilled

through a distillation column having a theoretical number of 15 plates. In the resulting distillate, however, 0.35 % of an impurity, α-chloro-orthoxylene remained (3 + 4-chloro-orthoxylene purity: 99.1 %). From Table 1 and Table 2, it is understood that α-chloro-orthoxylene would cause the purity reduction.

(Comparative Example 22) No treatment with minor metal followed by twice simple distillation:

45.2 g of the crude product obtained in Reference Example 3 was distilled, but the resulting distillate still contained 0.4% of an impurity, α-chloro-orthoxylene. The composition of the distillate is shown in Table 1 and Table 2 (3+4-chloro-orthoxylene purity: 98.9 %, yield: 17.8 g).

(Example 44) Selective decomposition (treatment with minor metal) followed by twice simple distillation with no pretreatment:

20.6 mg of $FeCl_3$ was added to 45.2 g of the crude product obtained in Reference Example 3, heated up to 100°C and stirred for 10 minutes, and complete decomposition of α-chloro-orthoxylene was confirmed. Then, this was cooled to room temperature and subjected twice to distillation under reduced pressure. A high-purity chloro-orthoxylene was obtained at a higher yield than in Comparative Example 22. From Tables 1 and 2, it is understood that, as compared with Comparative Example 21, this process is attained more simply and low cost and gives a product having a higher purity (3 + 4-chloro-orthoxylene purity: 99.3 %, yield: 19.9 g).

Table 1

| Entry No. | Decomposition Condition (remarks) | | | | |
|---|---|---|---|---|---|
| | Starting Material Mixture | Metal Used | Amount of Metal Used (w/w % vs crude product) | Reaction Temperature (°C) | Reaction Time (min) |
| Reference Example 2 | (starting material of Comparative Example 20) | | | | |
| Comparative Example 20 | Reference Example 2 | Zn | 70 | room temperature | 330 |
| Reference Example 1 | (starting material of Examples 37 to 43) | | | | |
| Example 37 | Reference Example 1 | Fe | 0.353 | 160 | 90 |
| Example 38 | Reference Example 1 | $FeCl_3$ | 0.15 | 160 | 10 |
| Example 39 | Reference Example 1 | $FeCl_3$ | 0.1 | 100 | 10 |
| Example 40 | Reference Example 1 | $FeCl_3$ | 0.06 | room temperature | 280 |
| Example 41 | Reference Example 1 | $FeCl_3$ | 0.06 | 100 | 20 |
| Example 42 | Reference Example 1 | $FeCl_3$ | 0.06 | 160 | 360 |
| Example 43 | Reference Example 1 | $AlCl_3$ | 0.12 | 100 | 10 |
| Comparative Example 21 | (15-stage distillation after base treatment before distillation) | | | | |
| Reference Example 3 | (starting material of Comparative Example 22, Example 44) | | | | |
| Comparative Example 22 | (no treatment with minor metal followed by twice simple distillation) | | | | |

Table 1 (continued)

| Entry No. | Decomposition Condition (remarks) | | | | |
|---|---|---|---|---|---|
| | Starting Material Mixture | Metal Used | Amount of Metal Used (w/w % vs crude product) | Reaction Temperature (°C) | Reaction Time (min) |
| Example 44 | Reference Example 3 | $FeCl_3$ | 0.046 | 100 | 10 |
| Example 44<br>Example 44 | (treatment with minor metal followed by twice simple distillation with no pretreatment) | | | | |

Table 2-1

| Entry No. | Starting Material and Reaction Mixture Composition (G.C. Area %)* | | | | |
|---|---|---|---|---|---|
| | OX | 2-CPX+ 2-CMX | 4-CMX | 5-CMX | 3-COX |
| Reference Example 2 | 0.0000 | 0.2823 | 0.1316 | 0.1481 | 4.6637 |
| Comparative Example 20 | 0.6080 | 0.2795 | 0.1292 | 0.1471 | 4.7737 |
| Reference Example 1 | 0.0000 | 0.2712 | 0.1298 | 0.1484 | 3.5163 |
| Example 37 | 0.0000 | 0.2619 | 0.1255 | 0.1475 | 3.3483 |
| Example 38 | 0.0000 | 0.2627 | 0.1273 | 0.1479 | 3.3817 |
| Example 39 | 0.0000 | 0.2660 | 0.1280 | 0.1497 | 3.3607 |
| Example 40 | 0.0000 | 0.2851 | 0.1320 | 0.1512 | 3.5838 |
| Example 41 | 0.0000 | 0.2644 | 0.1266 | 0.1491 | 3.3658 |
| Example 42 | 0.0000 | 0.2632 | 0.1264 | 0.1484 | 3.3505 |
| Example 43 | 0.0000 | 0.2660 | 0.1270 | 0.1497 | 3.4030 |
| Comparative Example 21 | 0.0000 | 0.2302 | 0.1086 | 0.1993 | 3.8687 |
| Reference Example 3 | 0.0000 | 0.2700 | 0.1240 | 0.1466 | 3.7835 |
| Comparative Example 22 | 0.0000 | 0.3043 | 0.1460 | 0.1770 | 5.0391 |
| Example 44 (after decomposition) | 0.0000 | 0.2628 | 0.1236 | 0.1491 | 3.6203 |
| Example 44 (after twice distillation) | 0.0000 | 0.3385 | 0.1695 | 0.1844 | 4.8319 |

Table 2-2

| Entry No. | Starting Material and Reaction Mixture Composition (G.C. Area %)* | | | | |
|---|---|---|---|---|---|
| | 4-COX | α-Cl | 3,5-DCOX | 3,6-DCOX | 4,5-DCOX |
| Reference Example 2 | 81.4961 | 0.5267 | 1.3350 | 2.1706 | 4.7543 |
| Comparative Example 20 | 82.1014 | 0.0000 | 1.3415 | 2.1394 | 4.7984 |
| Reference Example 1 | 76.6228 | 0.5020 | 3.1808 | 3.9816 | 6.0971 |
| Example 37 | 74.6588 | 0.0000 | 3.2387 | 4.0659 | 5.9943 |
| Example 38 | 76.3250 | 0.0000 | 3.3653 | 4.2345 | 5.8807 |
| Example 39 | 74.9738 | 0.0000 | 3.1328 | 3.9376 | 5.7711 |
| Example 40 | 78.2623 | 0.2141 | 2.8441 | 3.6172 | 5.0212 |
| Example 41 | 75.0516 | 0.0000 | 3.1740 | 3.9864 | 5.9996 |
| Example 42 | 74.9643 | 0.0000 | 3.2174 | 4.0456 | 6.4889 |

Table 2-2 (continued)

| Entry No. | Starting Material and Reaction Mixture Composition (G.C. Area %)* | | | | |
|---|---|---|---|---|---|
| | 4-COX | α-Cl | 3,5-DCOX | 3,6-DCOX | 4,5-DCOX |
| Example 43 | 75.1691 | 0.1049 | 3.1705 | 3.9814 | 5.9286 |
| Comparative Example 21 | 95.2419 | 0.3514 | 0.0000 | 0.0000 | 0.0000 |
| Reference Example 3 | 73.3190 | 0.5021 | 3.4140 | 4.1954 | 7.2375 |
| Comparative Example 22 | 93.8986 | 0.3993 | 0.0000 | 0.0000 | 0.0000 |
| Example 44 (after decomposition) | 72.7213 | 0.0000 | 3.4412 | 4.2452 | 7.0079 |
| Example 44 (after twice distillation) | 94.4757 | 0.0000 | 0.0000 | 0.0000 | 0.0000 |

Table 2-3

| Entry No. | Starting Material and Reaction Mixture Composition (G.C. Aera %)* | | | | 3-COX + 4-COX total purity | 4-COX selectivity |
|---|---|---|---|---|---|---|
| | 3,4-DCOX | α-Cl-3-COX | α-Cl-4-COX | others | | |
| Reference Example 2 | 3.0964 | 0.0675 | 0.6302 | 0.6975 | 86.2 | 94.6 |
| Comparative Example 20 | 3.0776 | 0.0000 | 0.0000 | 1.2122 | 86.9 | 94.5 |
| Reference Example 1 | 4.2004 | 0.1610 | 0.8883 | 0.3003 | 80.1 | 95.6 |
| Example 37 | 4.5383 | 0.0000 | 0.0000 | 3.6208 | 78.0 | 95.7 |
| Example 38 | 5.0912 | 0.0000 | 0.0000 | 1.1837 | 79.7 | 95.8 |
| Example 39 | 4.3876 | 0.0000 | 0.0000 | 3.8927 | 78.3 | 95.7 |
| Example 40 | 4.1886 | 0.1192 | 0.6638 | 0.9174 | 81.8 | 95.6 |
| Example 41 | 4.3007 | 0.0000 | 0.0000 | 3.5818 | 78.4 | 95.7 |
| Example 42 | 3.9862 | 0.0000 | 0.0000 | 3.4091 | 78.3 | 95.7 |
| Example 43 | 4.3530 | 0.0451 | 0.2513 | 3.0504 | 78.6 | 95.7 |
| Comparative Example 21 | 0.0000 | 0.0000 | 0.0000 | 0.0000 | 99.1 | 96.1 |
| Reference Example 3 | 4.9371 | 0.1815 | 1.1795 | 0.7098 | 77.1 | 95.1 |
| Comparative Example 22 | 0.0000 | 0.0000 | 0.0000 | 0.0357 | 98.9 | 94.9 |
| Example 44 (after decomposition) | 5.2684 | 0.0000 | 0.2155 | 2.8747 | 76.3 | 95.3 |
| Example 44 (after twice distillation) | 0.0000 | 0.0000 | 0.0000 | 0.0000 | 99.3 | 95.1 |

(*) OX = orthoxylene, CPX = chloro-paraxylene, CMX = chloro-metaxylene, COX =chloro-orthoxylene, α-Cl = α-chloro-orthoxylene, DCOX = dichloro-orthoxylene, α-Cl-3-COX = α-chloro-3-chloro-orthoxylene, α-Cl-4-COX = α-chloro-4-chloro-orthoxylene.

(Example 45)

(1) Preparation of Anhydrous Organic Reaction Liquid:

A 500-ml four-neck flask was equipped with a reflux condenser, a thermometer, a chlorine inlet tube, and a sampling tube. This was so designed that waste chlorine gas could be exhausted from the top of the reflux condenser and trapped in an aqueous sodium hydroxide solution. Heating it was effected in an oil bath.

84.8 g of orthoxylene (Sigma Aldrich Japan's first class grade chemical) that had been previously dried with Molecular Sieve 4A to have a water content of 20 ppm, 300 ml of 1,2-dichloroethane (Sigma Aldrich Japan's first class grade chemical) , and 33. 9 gof the fluorine-containingK-L-type zeolite (from Tosoh) that had been prepared in Example 2 and calcinated at 400°C for 1 hour and then cooled in a desiccator were put into the flask, and heated at 80°C with nitrogen gas being introduced thereinto through the chlorine gas inlet tube. After this was heated at 80°C, the nitrogen gas was exchanged for chlorine gas and the reaction was started. The chlorine gas flow rate was controlled to be 0.4 mol/hr. After reaction for 5 hours, a synthetic sample of 100% orthoxylene conversion was obtained.

(2) Filtration Test:

The anhydrous liquid-phase organic liquid produced in the above (1) was rapidly stirred in the flask, and a zeolite catalyst was suspended therein. 100 ml of the liquid was taken into a 200-ml Erlenmeyer flask. 2.3 g of water, corresponding to 0.3 times by weight of the zeolite amount in the collected sample liquid, was added to it, and stirred with a stirrer for 2 minutes so as to disperse water in the reaction liquid. Next, this was filtered under suction through a Nutsche filter device equipped with a 400-mesh filter (ASTM standard). The resulting filtrate was transparent, and no zeolite catalyst was found therein.

(Comparative Example 23)

In the same manner as in Example 45, the anhydrous liquid-phase organic reaction liquid produced in (1) was fully stirred in the flaks so as to suspend the zeolite catalyst therein. Then, 100 ml of the liquid was taken into a 200-ml Erlenmeyer flask. Thus collected, it was directly filtered under suction through a Nutsche filter device equipped with a 400-mesh filter (ASTM standard). The resulting filtrate was cloudy and milky, and this was contaminated with some zeolite catalyst remaining therein.

INDUSTRIAL APPLICABILITY

The invention ensures para-selectivity in producing di-substituted halobenzene derivatives from benzene or benzene derivatives, and ensures 1,2,4-tri-selective halogenation in producing tri-substituted halobenzene derivatives from them.

## Claims

1. A process for halogenation of benzene or benzene derivatives, which comprises using a fluorine-containing zeolite catalyst in halogenation of benzene or benzene derivatives.

2. A process for halogenation of benzene or benzene derivatives, which comprises using a zeolite catalyst having the crystal size of at most 100 nm in halogenation of benzene or benzene derivatives.

3. The process for halogenation of benzene or benzene derivatives as claimed in claim 1 or 2, wherein the zeolite is an L-type zeolite.

4. The process for halogenation of benzene or benzene derivatives as claimed in claim 3, wherein the catalyst is an L-type zeolite having a water content of at most 1. 0 % by weight.

5. The process for halogenation of benzene or benzene derivatives as claimed in claim 3 or 4, wherein an L-type zeolite previously calcinated at a temperature not lower than 200°C is used.

6. The process for halogenation of benzene or benzene derivatives as claimed in any one of claims 3 to 5, wherein an L-type zeolite previously dried at a temperature not lower than 100°C is used, not exposed at all to air before use.

7. The process for halogenation of benzene or benzene derivatives as claimed in any one of claims 1 to 6, wherein the halogenation is effected in the presence of a solvent.

8. The process for halogenation of benzene or benzene derivatives as claimed in claim 7, wherein the solvent is a halogenated compound.

9. The process for halogenation of benzene or benzene derivatives as claimed in claim 8, wherein the halogenated compound is a dihalogenoethane or dihalogenopropane.

10. The process for halogenation of benzene or benzene derivatives as claimed in any one of claims 1 to 9, wherein the halogenation is chlorination.

11. The process for halogenation of benzene or benzene derivatives as claimed in claim 10, wherein the chlorination is effected with a previously-diluted chlorine gas.

12. The process for halogenation of benzene or benzene derivatives as claimed in claim 11, wherein the chlorination is effected with a chlorine gas previously diluted with an inert gas.

13. The process for halogenation of benzene or benzene derivatives as claimed in claim 11 or 12, wherein the concentration of the diluted chlorine gas is from 5 mol% to 99 mol%.

14. The process for halogenation of benzene or benzene derivatives as claimed in any one of claims 10 to 13, wherein the chlorination is effected in the presence of a solvent, chlorinated compound.

15. The process for halogenation of benzene or benzene derivatives as claimed in claim 14, wherein the chlorinated compound is dichloroethane or dichloropropane.

16. The process for halogenation of benzene or benzene derivatives as claimed in any one of claims 1 to 15, wherein the water content of the starting material, benzene or benzene derivative to be halogenated is at most 100 ppm.

17. The process for halogenation of benzene or benzene derivatives as claimed in any one of claims 1 to 16, wherein the benzene or benzene derivative is further halogenated after 100 % conversion thereof.

18. The process for halogenation of benzene or benzene derivatives as claimed in any one of claims 1 to 17, wherein the benzene or benzene derivative is a di-substituted benzene derivative that has ortho or meta-aromatic compound.

19. The process for halogenation of benzene or benzene derivatives as claimed in claim 18, wherein the benzene or benzene derivative is orthoxylene and the halobenzene derivative obtained is 4-chloro-orthoxylene.

20. The process for halogenation of benzene or benzene derivatives as claimed in claim 18 or 19, wherein orthoxylene is converted to at least 95 %.

21. A process for halogenation of benzene or benzene derivatives, wherein a halide of a benzene or benzene derivative is obtained according to the process of any one of claims 1 to 20, then from 0.001 to 10 % by weight of at least one selected from metals and metal salts, relative to the total of the benzene or benzene derivative halide and an impurity, α-haloalkylbenzene derivative, is added to it, and this is then distilled.

22. The process for halogenation of benzene or benzene derivatives as claimed in claim 21, wherein the metal is at least one selected from iron, aluminium, antimony, gallium, zirconium, tin and boron.

23. The process for halogenation of benzene or benzene derivatives as claimed in claim 21 or 22, wherein the distillation is effected in the presence of a solvent.

24. The process for halogenation of benzene or benzene derivatives as claimed in claim 21, wherein the benzene or benzene derivative halide is a haloalkylbenzene derivative of the following general formula (I) and the impurity is an α-haloalkylbenzene derivative of the following general formula (II):

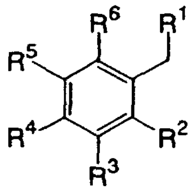

(I)

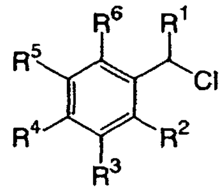

(II)

(in formula (I), the groups $R^1$ to $R^6$ are independent of each other; $R^1$ represents a hydrogen atom, or an alkyl group having from 1 to 4 carbon atoms; $R^2$ to $R^6$ each represent a hydrogen atom, a halogen atom, or an alkyl group having from 1 to 5 carbon atoms, and at least one or more of these groups are any of a halogen atom and an alkyl group having from 1 to 5 carbon atoms; in formula (II), the groups $R^1$ to $R^6$ are independent of each other; $R^1$ represents a hydrogen atom, or an alkyl group having from 1 to 4 carbon atoms; $R^2$ to $R^6$ each represent a hydrogen atom, a halogen atom, an alkyl group having from 1 to 5 carbon atoms, or an α-haloalkyl group having from 1 to 5 carbon atoms, and at least one or more of these groups are any of a halogen atom and a hydrogen atom.)

**25.** The process for halogenation of benzene or benzene derivatives as claimed in claim 21, wherein the benzene or benzene derivative halide is a haloalkylbenzene derivative of the following general formula (I) and the impurity is an α-haloalkylbenzene derivative of the following general formula (II):

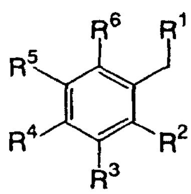

(I)

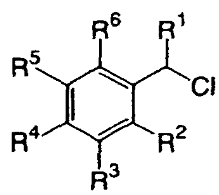

(II)

(in formula (I), the groups $R^1$ to $R^6$ are independent of each other; $R^1$ represents a hydrogen atom; $R^2$ to $R^6$ each represent a hydrogen atom, a chlorine atom, or a methyl group, and at least one of these groups is a chlorine atom or a methyl group; in formula (II), the groups $R^1$ to $R^6$ are independent of each other; $R^1$ represents a hydrogen atom; $R^2$ to $R^6$ each represent a hydrogen atom, a chlorine atom, a methyl group or an α-chloromethyl group, and at least one of these groups is a chlorine atom, a hydrogen atom or a methyl group.)

**26.** The process for halogenation of benzene or benzene derivatives as claimed in any one of claims 1 to 25, wherein, in the step of separating zeolite from the zeolite-containing, anhydrous liquid-phase reaction liquid after the reaction, water is added to the anhydrous liquid-phase reaction liquid so as to aggregate the dispersed zeolite, and then this is introduced into a solid-liquid separator so as to separate the zeolite catalyst from it.

Fig. 1

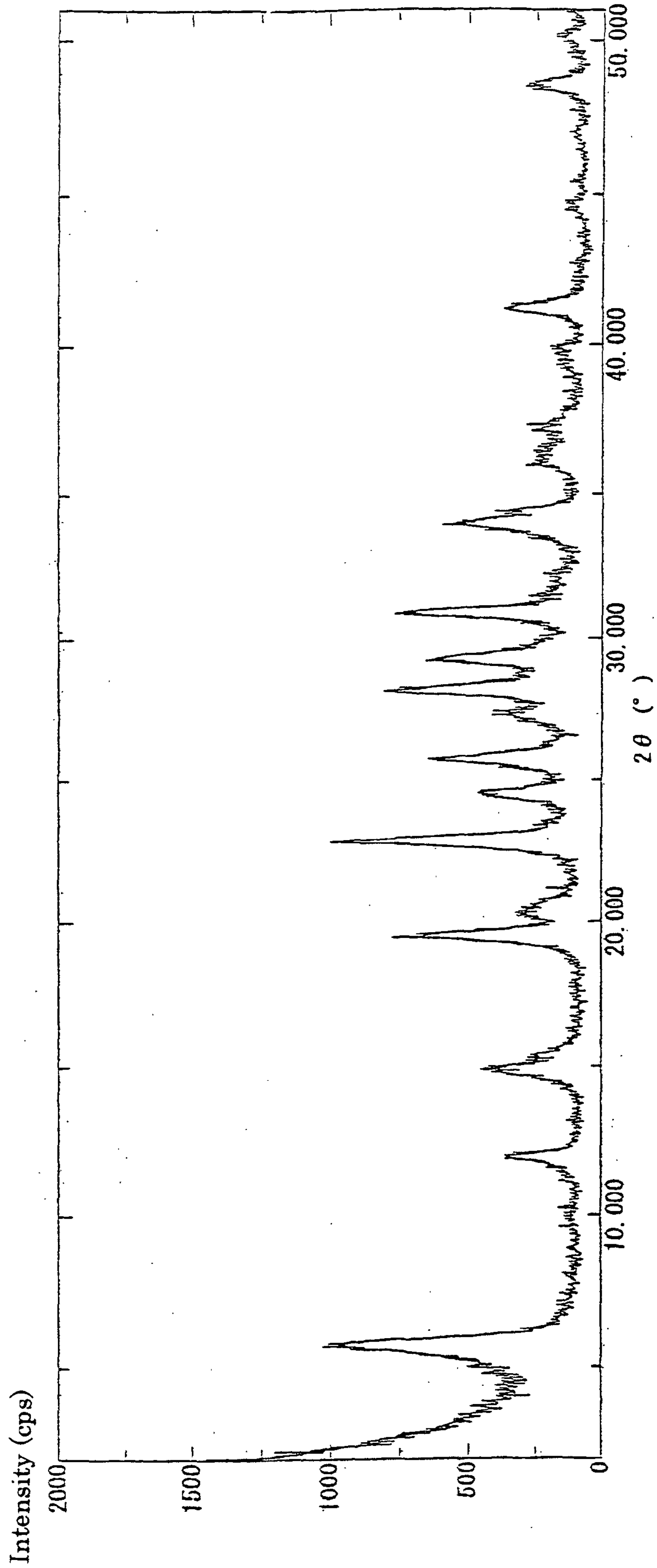
Intensity (cps)
2000
1500
1000
500
0
10.000
20.000
30.000
40.000
50.000
2θ (°)

Fig. 2

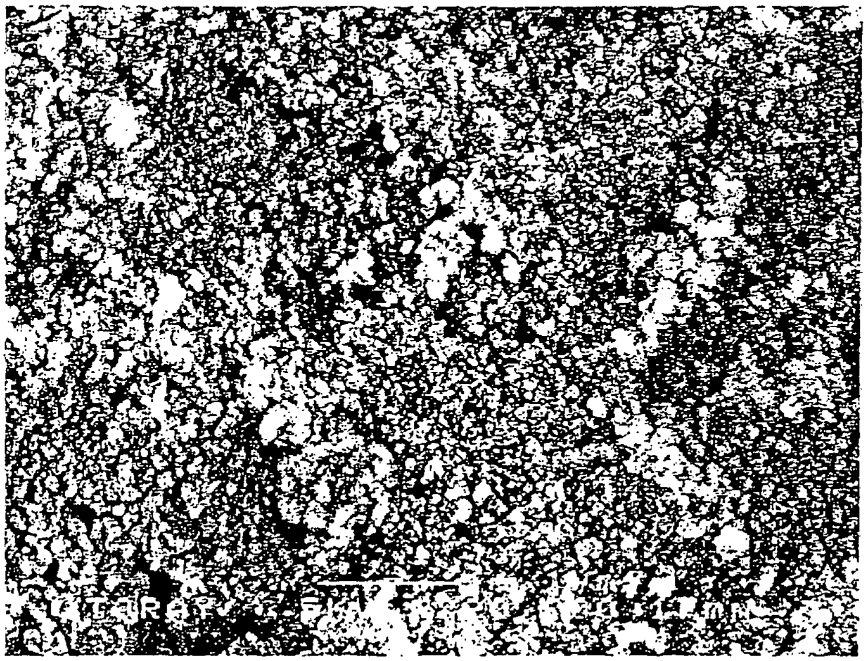

Fig. 3

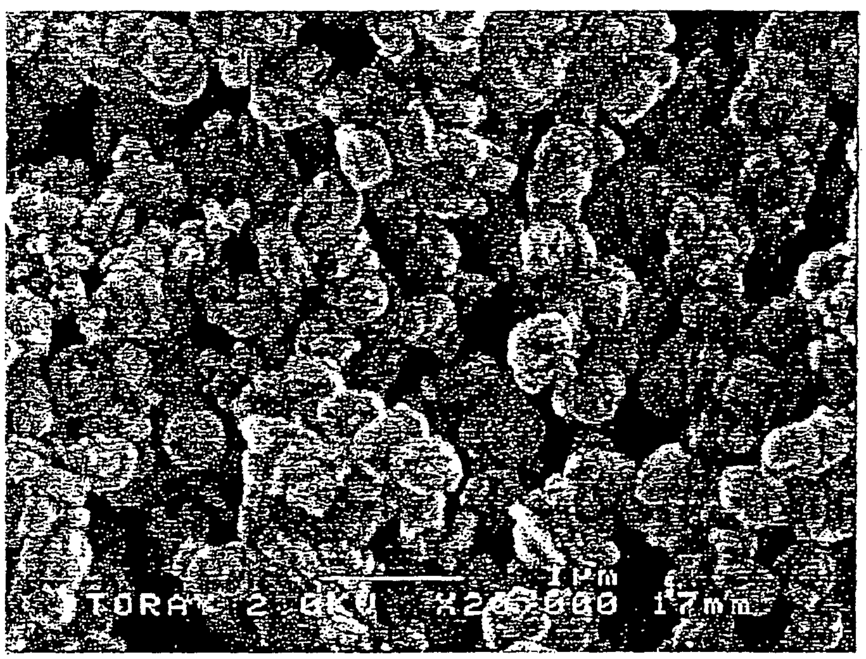

Fig. 4

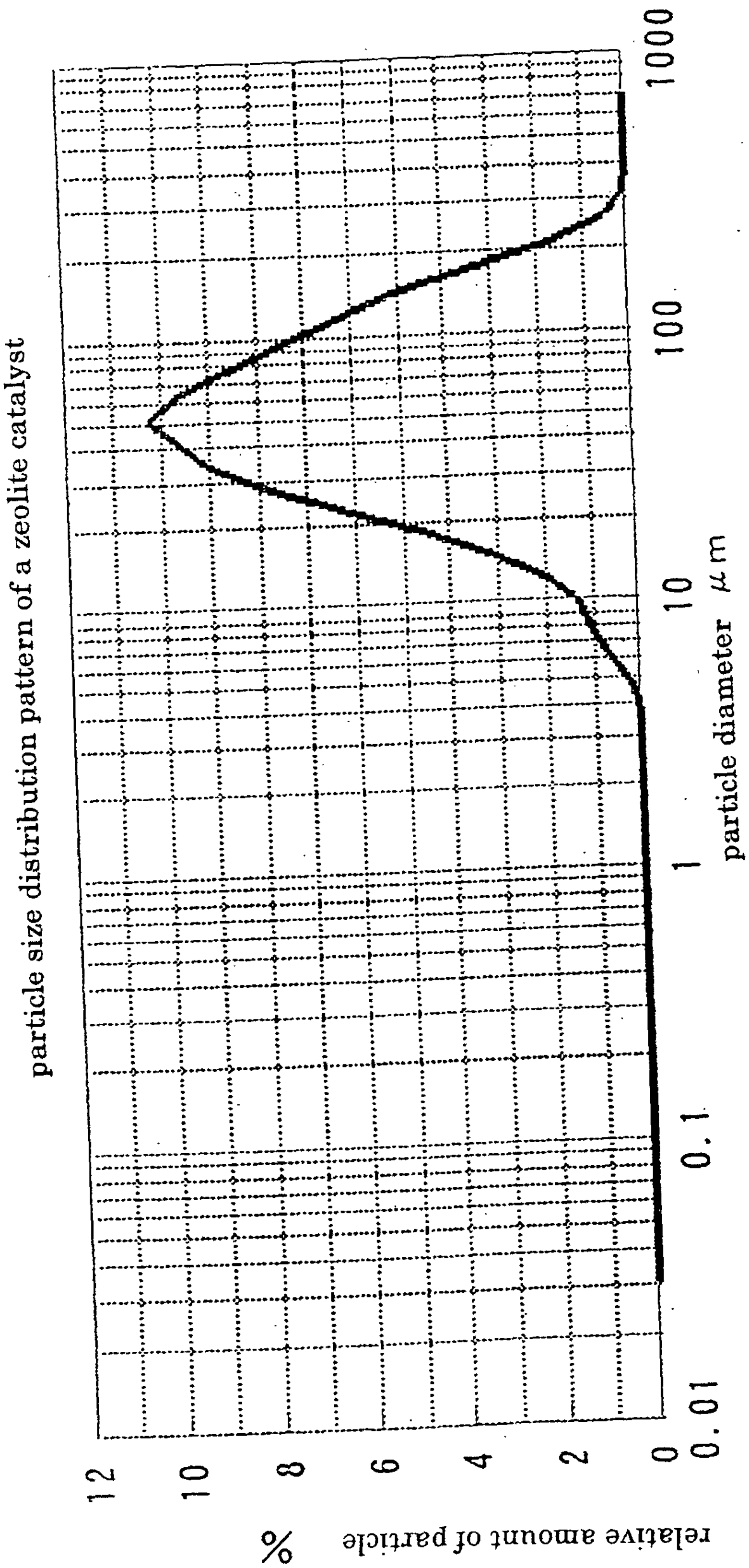
particle size distribution pattern of a zeolite catalyst
particle diameter μm
0.01
0.1
1
10
100
1000
relative amount of particle %
0
2
4
6
8
10
12

Fig. 5

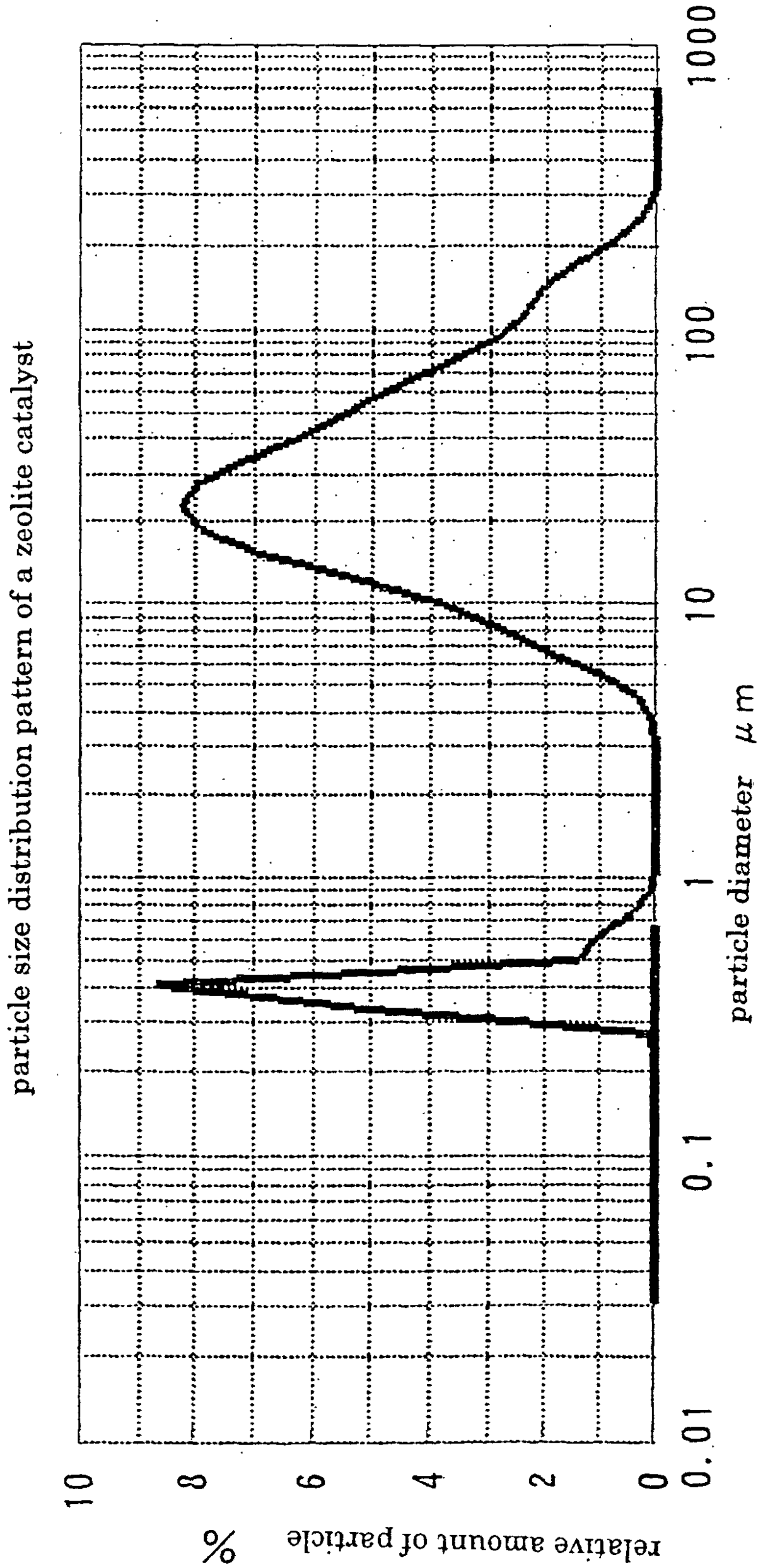
particle size distribution pattern of a zeolite catalyst
particle diameter μm
0.01
0.1
1
10
100
1000
relative amount of particle %
0
2
4
6
8
10

Fig. 6

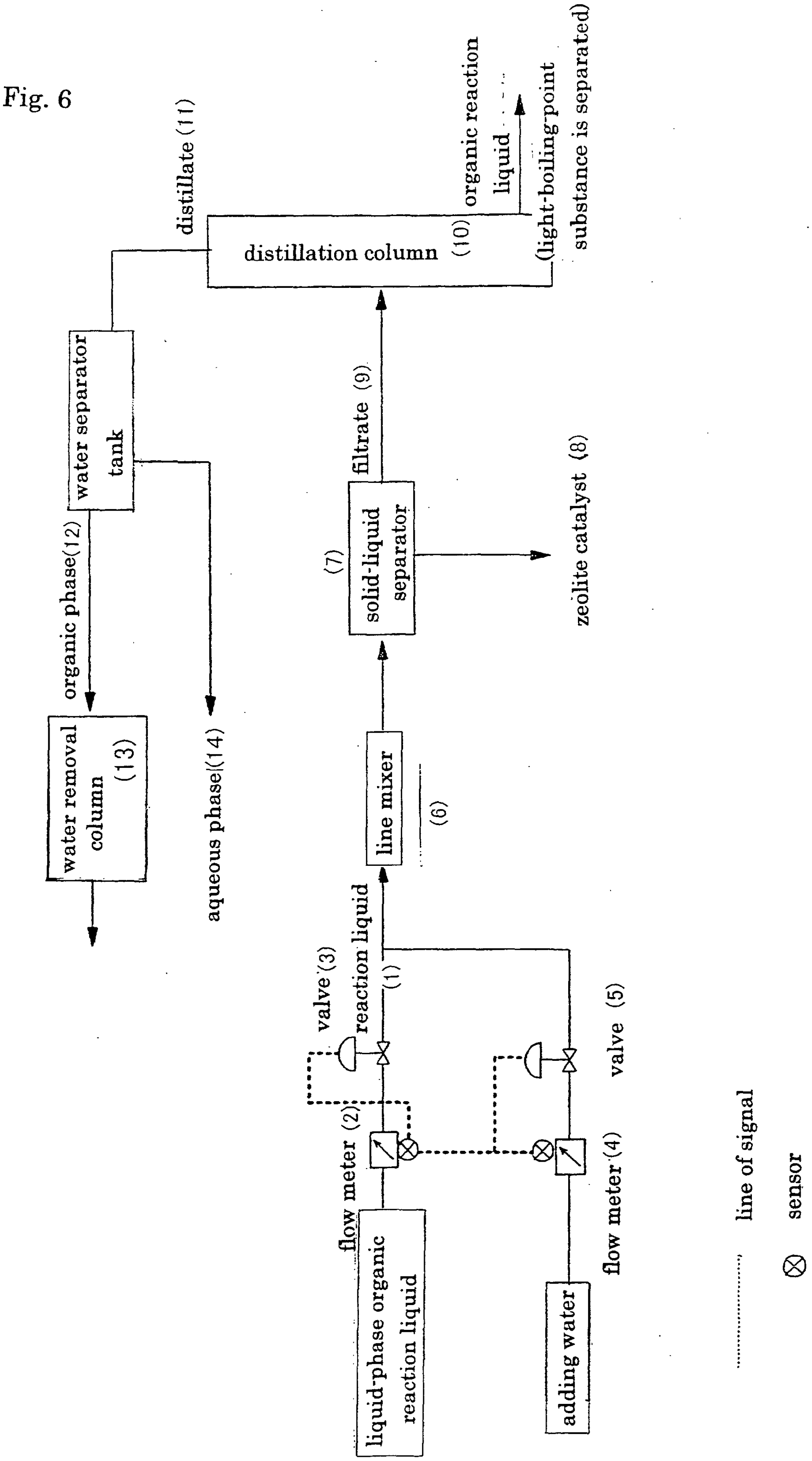
distillate (11)
distillation column
(10)
organic reaction liquid
(light-boiling-point substance is separated)
water separator tank
organic phase (12)
water removal column (13)
aqueous phase (14)
filtrate (9)
(7)
solid-liquid separator
zeolite catalyst (8)
line mixer
(6)
valve (3)
reaction liquid (1)
flow meter (2)
valve (5)
flow meter (4)
liquid-phase organic reaction liquid
adding water
line of signal
sensor

Fig. 7

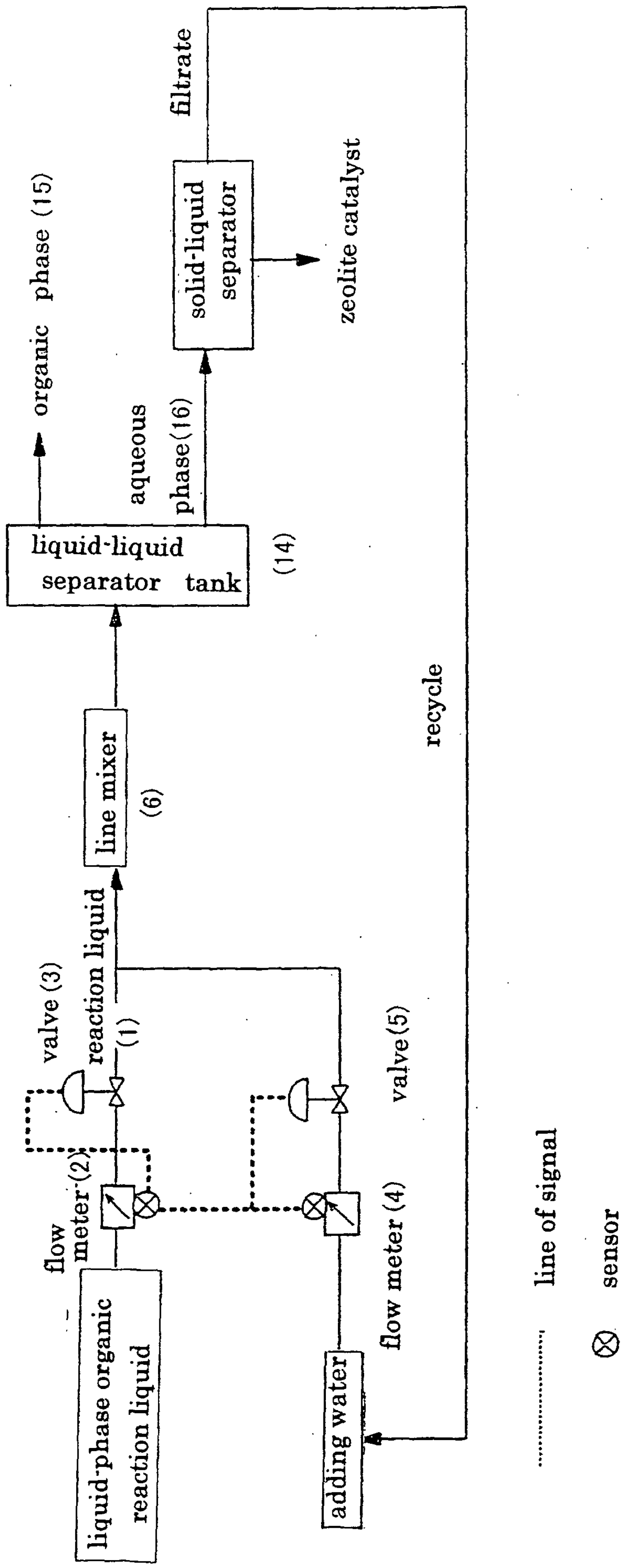
liquid-phase organic reaction liquid
flow meter (2)
valve (3)
reaction liquid (1)
line mixer (6)
liquid-liquid separator tank
(14)
organic phase (15)
aqueous phase(16)
solid-liquid separator
zeolite catalyst
filtrate
recycle
valve(5)
flow meter (4)
adding water
line of signal
sensor

## INTERNATIONAL SEARCH REPORT

International application No.
PCT/JP03/06464

A. CLASSIFICATION OF SUBJECT MATTER
Int.Cl7 C07C25/02, 25/125, 17/12, C07B61/00

According to International Patent Classification (IPC) or to both national classification and IPC

B. FIELDS SEARCHED

Minimum documentation searched (classification system followed by classification symbols)
Int.Cl7 C07C25/02, 25/125, 17/12, C07B61/00

Documentation searched other than minimum documentation to the extent that such documents are included in the fields searched

Electronic data base consulted during the international search (name of data base and, where practicable, search terms used)
WPI(DIALOG)

C. DOCUMENTS CONSIDERED TO BE RELEVANT

| Category* | Citation of document, with indication, where appropriate, of the relevant passages | Relevant to claim No. |
|---|---|---|
| X<br>A | JP 6-321822 A (Nippon Light Metal Co., Ltd.), 22 November, 1994 (22.11.94), Claims; Par. Nos. [0006] to [0008], [0012] (Family: none) | 1,3,10-13, 16-20<br>2,4-9,14,15, 21-26 |
| X<br>A | EP 154236 A1 (IHARA CHEMICAL INDUSTRY CO., LTD.), 11 September, 1985 (11.09.85), Claims; page 7, lines 4 to 23 & JP 60-188333 A & US 4831199 A & US 4942268 A | 1,3,7,10-13, 17-20<br>2,4-6,8,9, 14-16,21-26 |
| X<br>A | JP 2000-128811 A (Mitsubishi Chemical Corp.), 09 May, 2000 (09.05.00), Claims; examples (Family: none) | 2-5,7-15, 17-20<br>1,6,16,21-26 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 05 August, 2003 (05.08.03) | 26 August, 2003 (26.08.03) |
| Name and mailing address of the ISA/<br>Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP03/06464

C (Continuation). DOCUMENTS CONSIDERED TO BE RELEVANT

| Category* | Citation of document, with indication, where appropriate, of the relevant passages | Relevant to claim No. |
|---|---|---|
| X<br>A | JP 2000-128810 A (Mitsubishi Chemical Corp.),<br>09 May, 2000 (09.05.00),<br>Claims; examples<br>(Family: none) | 2-15,17-20<br>1,16,21-26 |
| A | US 5993607 A (Elf Atochem, S.A.),<br>30 November, 1999 (30.11.99),<br>& JP 10-330300 A & EP 870748 A1 | 1-26 |

Form PCT/ISA/210 (continuation of second sheet) (July 1998)

## INTERNATIONAL SEARCH REPORT

International application No.
PCT/JP03/06464

**Box I Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:

The matter common to claims 1-26 is "a process for halogenating benzene or benzene derivatives by using a zeolite as the catalyst". As stated by the applicant in the description, however, this process is a publicly known matter disclosed in Japanese Patent Laid-Open Nos. Sho 59-163329, Sho 57-77631, and Hei 3-81234, thus being not a special technical feature within the meaning of PCT Rule 13.2.

Further, there is no other common matter considered as a special technical feature. Thus, claims 1-26 are not considered as relating to a group of inventions so linked as to form a single general inventive concept.

(continued to extra sheet)

1. ☒ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.

☒ No protest accompanied the payment of additional search fees.

Form PCT/ISA/210 (continuation of first sheet (1)) (July 1998)

**INTERNATIONAL SEARCH REPORT**

International application No.
PCT/JP03/06464

Continuation of Box No.II of continuation of first sheet(1)

Therefore, claim 1 relates to an invention characterized by using a fluorine-containing zeolite, and claim 2 relates to an invention characterized by using a zeolite having a crystallite diameter of 100 nm or below, claims 3-26 relating to inventions involving the above two different inventions.